(19) Europäisches Patentamt
European Patent Office
Office européen des brevets

(11) Publication number: **0 659 751 A1**

(12) # EUROPEAN PATENT APPLICATION

(21) Application number: **94120185.7**

(51) Int. Cl.[6]: **C07D 403/06**, A61K 31/41

(22) Date of filing: **20.12.94**

(30) Priority: **22.12.93 JP 324663/93**

(43) Date of publication of application: **28.06.95 Bulletin 95/26**

(84) Designated Contracting States: **AT BE CH DE DK ES FR GB GR IE IT LI LU NL PT SE**

(71) Applicant: **TAKEDA CHEMICAL INDUSTRIES, LTD.**
**1-1, Doshomachi 4-chome**
**Chuo-ku,**
**Osaka 541 (JP)**

(72) Inventor: **Itoh, Katsumi**
**18-12, Kofudai 6-chome,**
**Toyono-cho**
**Toyono-gun,**
**Osaka 563-01 (JP)**
Inventor: **Tasaka, Akihiro**
**9-20-102, Yamadahigashi 2-chome**
**Suita,**
**Osaka 565 (JP)**
Inventor: **Matsushita, Yoshihiro**
**3-29, Minamimukonoso 5-chome**
**Amagasaki,**
**Hyogo 661 (JP)**

(74) Representative: **von Kreisler, Alek, Dipl.-Chem. et al**
**Patentanwälte**
**von Kreisler-Selting-Werner**
**Bahnhofsvorplatz 1 (Deichmannhaus)**
**D-50667 Köln (DE)**

(54) **Optically active azole derivatives, their production and use.**

(57) A process for preparing a compound of the formula (IV) or a salt thereof,

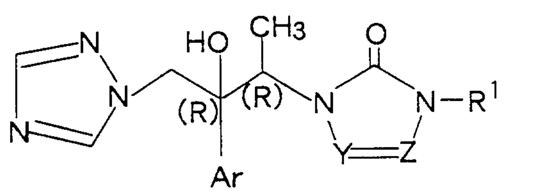

(IV)

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar is a halogenated phenyl group, and (R) shows that the carbon atom marked with (R) has (R)-configuration, in a short period, by simple processes and with high yield and purity; and an azole compound of the formula (IV'),

EP 0 659 751 A1

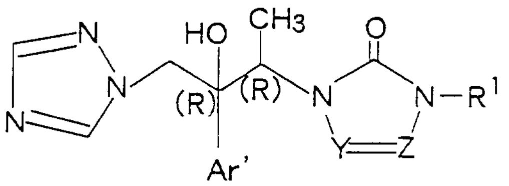

(IV')

, wherein Ar' is a monofluorophenyl group, and the other symbols have the same meanings as above, or a salt thereof, which are useful for an antifungal therapeutic agent.

BACKGROUND OF THE INVENTION

1. Field of the Invention

The present invention relates to optically active azole derivatives, their production and use. The optically active azole derivatives of the invention are useful for an anti-fungal therapeutic agent (especially in mycosis).

2. Description of the Related Art

A method for manufacturing optically active azole derivatives having a triazolone group by reacting a triazolone derivative with an oxirane-triazole derivative in the presence of sodium hydride is described in EPA-567,982.

It has been demanded to develop a practical method for the production of optically active azole derivatives in a short time, by simple processes and with high yield and purity.

Various compounds have been reported already as anti-fungal agents (for example, EPA-122,693, EPA-122,056 and EPA-332,387). These compounds are, however, not satisfactory in their therapeutic effects from the viewpoint of anti-fungal activity, antifungal spectrum, side effect and pharmacokinetics.

Conventional antifungal agents do not exhibit sufficient therapeutic effect and, in addition, there are many problems on side effects, pharmacokinetics, superinfection and acquisition of drug-resistance.

For solving such problems, it would be clear that compounds having higher safety, better absorption in vivo and more potent antifungal activity are desired as therapeutic agents.

SUMMARY OF THE INVENTION

The present invention provides:

A) a process for preparing a compound of the formula (IV) or a salt thereof:

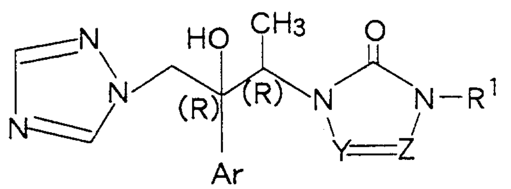

(IV)

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar is a halogenated phenyl group, and (R) shows that the carbon atom marked with (R) has (R)-configuration,

which comprises reacting a compound of the formula (I) or a salt thereof:

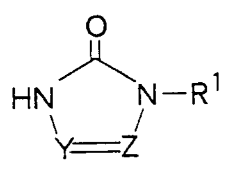

(I)

, wherein the symbols have the same meanings as defined above,

with a compound of the formula (III):

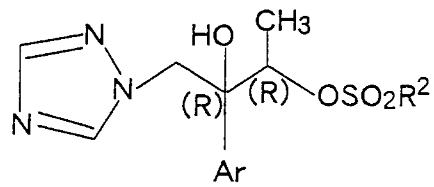

(III)

, wherein $R^2$ is a lower alkyl group or an optionally substituted phenyl group and the other symbols have the same meanings as defined above,

B) a process for preparing a compound of the formula (IV) or a salt thereof:

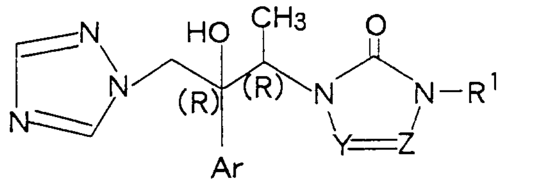

(IV)

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar is a halogenated phenyl group, and (R) shows that the carbon atom marked with (R) has (R)-configuration,

which comprises reacting a compound of the formula (I) or a salt thereof:

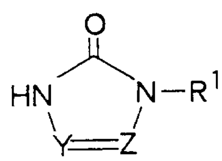

(I)

, wherein the symbols have the same meanings as defined above,

with a compound of the formula (II):

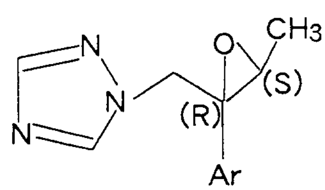

(II)

, wherein (S) shows that the carbon atom marked with (S) has (S)-configuration and the other symbols have the same meanings as defined above, in the presence of an alkali metal carbonate,

C) an azole compound of the formula (IV'):

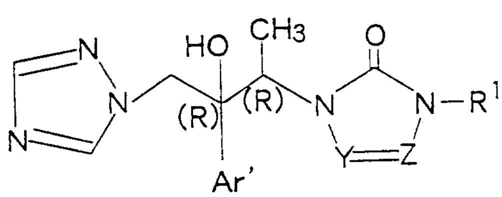

(IV')

, wherein $R^1$ is an optionally substituted aliphatic or aromatic hydrocarbon residue or an optionally

substituted aromatic heterocyclic group, one of Y and Z is a nitrogen atom and the other is a methine group which may be optionally substituted with a lower alkyl group, Ar' is a monofluorophenyl group, and (R) shows that the carbon atom marked with (R) has (R)-configuration,

or a salt thereof and

D) an antifungal composition comprising the above mentioned compound (IV') or a pharmaceutically acceptable salt thereof, and a carrier, excipient or diluent.

DETAILED DESCRIPTION OF THE PREFERRED EMBODIMENT

The definition for $R^1$ in the formulae (I), (IV) and (IV') includes an optionally substituted aliphatic or aromatic hydrocarbon residue and an optionally substituted aromatic heterocyclic residue. $R^1$ is preferably an optionally substituted aromatic hydrocarbon residue, more preferably an optionally substituted phenyl group.

Examples of the optionally substituted aliphatic hydrocarbon groups include optionally substituted alkyl, cycloalkyl, alkenyl or alkynyl groups.

Examples of the alkyl groups are straight or branched alkyl groups having 1 to 12 carbon atoms such as methyl, ethyl, n-propyl, isopropyl, n-butyl, isobutyl, sec-butyl, tert-butyl, heptyl, octyl, nonyl, decyl or dodecyl, among which lower alkyl groups having 1 to 4 carbon atoms (e.g., methyl, ethyl, n-propyl, isopropyl, n-butyl, isobutyl, sec-butyl and tert-butyl) are preferred.

Examples of the cycloalkyl groups are cycloalkyl groups having 3 to 8 carbon atoms such as cyclopropyl, cyclobutyl, cyclopentyl, cyclohexyl, cycloheptyl and cyclooctyl, among which cycloalkyl groups having 3 to 6 carbon atoms (e.g., cyclopropyl, cyclobutyl, cyclopentyl and cyclohexyl) are preferred.

Examples of the alkenyl groups are alkenyl groups having 2 to 4 carbon atoms such as vinyl, propenyl and butenyl, among which alkenyl groups having 2 to 3 carbon atoms (e.g., vinyl and propenyl) are preferred.

Examples of the alkynyl groups are alkynyl groups having 2 to 4 carbon atoms such as ethynyl, propynyl and butynyl, among which alkynyl groups having 2 to 3 carbon atoms (e.g., ethynyl, propynyl) are preferred.

Examples of the optionally substituted aromatic hydrocarbon residues are optionally substituted aryl groups having 6 to 14 carbon atoms. Examples of the aryl groups are phenyl, naphthyl, biphenylyl, anthryl or indenyl, among which aryl groups having 6 to 10 carbon atoms (e.g., phenyl or naphthyl) are preferred.

Examples of the optionally substituted aromatic heterocyclic groups are optionally substituted aromatic heterocyclic groups having at least one hetero atom selected from a nitrogen atom, sulfur atom and oxygen atom. The aromatic heterocyclic groups may be fused with benzene or 5- or 6-membered heterocyclic rings. Examples of the aromatic heterocyclic groups are non-fused aromatic heterocyclic groups such as imidazolyl, triazolyl, tetrazolyl, pyrazolyl, pyridyl, thiazolyl, thiadiazolyl, thienyl, furyl, pyrrolyl, pyrazinyl, pyrimidinyl, oxazolyl and isoxazolyl, and fused aromatic heterocyclic groups such as benzimidazolyl, imidazopyrimidinyl, imidazopyridinyl, imidazopyrazinyl, imidazopyridazinyl, benzothiazolyl, quinolyl, isoquinolyl, quinazolinyl and indolyl, among which optionally substituted 5- or 6-membered aromatic heterocyclic groups (e.g., imidazolyl, triazolyl, thiazolyl, thiadiazolyl, thienyl, furyl, pyridyl and pyrimidinyl) having 1 to 3 hetero atoms optionally selected from a nitrogen atom, sulfur atom and oxygen atom are preferred.

Examples of the substituents for the optionally substituted aliphatic or aromatic hydrocarbon residues and the optionally substituted aromatic heterocyclic groups shown by $R^1$ are hydroxy group, optionally esterified carboxy group (e.g., carboxy, or alkoxycarbonyl having 1 to 6 carbon atoms such as methoxycarbonyl, ethoxycarbonyl and butoxycarbonyl), nitro group, amino group, acylamino group (e.g., alkanoylamino group having 1 to 10 carbon atoms such as acetylamino, propionylamino and butyrylamino), amino group substituted with mono- or di- alkyl group (e.g., methylamino, dimethylamino, diethylamino and dibutylamino), optionally substituted 5- or 6-membered heterocyclic group (e.g., pyrrolidinyl, morpholino, piperidino, piperazinyl, 4-benzyl-1-piperazinyl, 4-acetyl-1-piperazinyl, 4-(4-methoxyphenyl)-1-piperazinyl, 4-(4-trifluoromethoxyphenyl)-1-piperazinyl, 4-[4-(1,1,2,2-tetrafluoroethoxy)phenyl]-1-piperazinyl, 4-[4-(2,2,2-trifluoroethoxy)phenyl]-1-piperazinyl, 4-[4-(2,2,3,3,3-pentafluoropropoxy)phenyl]-1-piperazinyl, 4-(4-trifluoromethyl)phenyl-1-piperazinyl, 4-[4-(2,2,3,3-tetrafluoropropoxy)phenyl]-1-piperazinyl, pyrazolizinyl and perhydroazepinyl), alkoxy group having 1 to 6 carbon atoms (e.g., methoxy, ethoxy and butoxy), halogen (e.g., fluorine, chlorine and bromine), haloalkyl group having 1 to 6 carbon atoms (e.g., trifluoromethyl, dichloromethyl and trifluoroethyl), haloalkoxy group having 1 to 6 carbon atoms (e.g., trifluoromethoxy, 1,1,2,2-tetrafluoroethoxy, 2,2,2-trifluoroethoxy, 2,2,3,3-tetrafluoropropoxy, 2,2,3,3,3-pentafluoropropoxy, 2,2,3,3,4,4,5,5-octafluoropentoxy and 2-fluoroethoxy), oxo group, thioxo group, mercapto group, alkylthio

group having 1 to 6 carbon atoms (e.g., methylthio, ethylthio and butylthio), alkylsulfonyl group having 1 to 6 carbon atoms (e.g., methanesulfonyl, ethanesulfonyl and butanesulfonyl) and alkanoyl group having 1 to 10 carbon atoms (e.g., acetyl, formyl, propionyl and butyryl). The number of the substituents described above is preferably 1 to 3 and more preferably 1 or 2.

More preferably, $R^1$ is an aryl group having 6 to 10 carbon atoms optionally substituted with a haloalkoxy group having 1 to 6 carbon atoms. Most preferably, $R^1$ is a phenyl group substituted with a haloalkoxy group having 1 to 6 carbon atoms.

In the formulae (I), (IV) and (IV'), examples of the lower alkyl groups in "the methine group which may be optionally substituted with a lower alkyl group represented by Y or Z" include a straight or branched alkyl group having 1 to 4 carbon atoms (e.g., methyl, ethyl, n-propyl, isopropyl, n-butyl, isobutyl, sec-butyl and tert-butyl), among which methyl is preferable. Examples of the methine group represented by Y and Z which may be optionally substituted with a lower alkyl group include methine, ethylidine, propylidine, butylidine and the like. Among them, methine and ethylidine are preferable.

In the formulae (II), (III) and (IV), examples of the halogenated phenyl groups represented by Ar include phenyl groups substituted with 1 to 3 halogen atoms selected from the group of fluorine, chlorine, bromine and iodine, specifically 2,4-difluorophenyl, 2,4-dichlorophenyl, 4-chlorophenyl, 4-fluorophenyl, 2-chlorophenyl, 2-fluorophenyl, 2-fluoro-4-chlorophenyl, 2-chloro-4-fluorophenyl, 2,4,6-trifluorophenyl, 4-bromophenyl and the like, among which a phenyl group substituted with 1 or 2 fluorine atoms is preferable. More preferably, 2-fluorophenyl, 4-fluorophenyl or 2,4-difluorophenyl are used.

In the formula (IV'), the monofluorophenyl group represented by Ar' is preferably 2-fluorophenyl and 4-fluorophenyl.

In the formula (III), examples of the lower alkyl groups represented by $R^2$ include straight or branched alkyl groups having 1 to 4 carbon atoms (e.g., methyl, ethyl, n-propyl, isopropyl, n-butyl, isobutyl, sec-butyl and tert-butylmethyl), among which methyl is preferable. Examples of optionally substituted phenyl groups represented by $R^2$ include phenyl, p-tolyl, p-chlorophenyl, p-nitrophenyl and the like, among which p-tolyl is preferable.

Examples of preferable compounds represented by the formula (IV') include:

2-[(1R,2R)-2-(2-fluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone,

2-[(1R,2R)-2-(2-fluorophenyl)-2-hydroxy-1-methyl-3-(1H-1,2,4-triazol-1-yl)propyl]-4-(4-trifluoromethylphenyl)-3(2H,4H)-1,2,4-triazolone,

2-[(1R,2R)-2-(2-fluorophenyl)-2-hydroxy-1-methyl-3-(1H-1,2,4-triazol-1-yl)propyl]-4-(4-trifluoromethoxyphenyl)-3(2H,4H)-1,2,4-triazolone,

2-[(1R,2R)-2-(2-fluorophenyl)-2-hydroxy-1-methyl-3-(1H-1,2,4-triazol-1-yl)propyl]-4-[4-(2,2,2-trifluoroethoxy)phenyl]-3-(2H,4H)-1,2,4-triazolone,

2-[(1R,2R)-2-(2-fluorophenyl)-2-hydroxy-1-methyl-3-(1H-1,2,4-triazol-1-yl)propyl]-4-[4-(1,1,2,2-tetrafluoroethoxy)phenyl]-3(2H,4H)-1,2,4-triazolone,

2-[(1R,2R)-2-(2-fluorophenyl)-2-hydroxy-1-methyl-3-(1H-1,2,4-triazol-1-yl)propyl]-5-methyl-4-[4-(1,1,2,2-tetrafluoroethoxy)phenyl]-3(2H,4H)-1,2,4-triazolone,

4-[(1R,2R)-2-(2-fluorophenyl)-2-hydroxy-1-methyl-3-(1H-1,2,4-triazol-1-yl)propyl]-2-[4-(1,1,2,2-tetrafluoroethoxy)phenyl]-3(2H,4H)-1,2,4-triazolone,

4-[(1R,2R)-2-(2-fluorophenyl)-2-hydroxy-1-methyl-3-(1H-1,2,4-triazol-1-yl)propyl]-2-[4-(2,2,3,3-tetrafluoropropoxy)phenyl]-3(2H,4H)-1,2,4-triazolone,

2-[(1R,2R)-2-(4-fluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone,

2-[(1R,2R)-2-(4-fluorophenyl)-2-hydroxy-1-methyl-3-(1H-1,2,4-triazol-1-yl)propyl]-4-[4-(1,1,2,2-tetrafluoro ethoxy)phenyl]-3(2H,4H)-1,2,4-triazolone,

2-[(1R,2R)-2-(4-fluorophenyl)-2-hydroxy-1-methyl-3-(1H-1,2,4-triazol-1-yl)propyl]-5-methyl-[4-(1,1,2,2-tetrafluoroethoxy)phenyl]-3(2H,4H)-1,2,4-triazolone,

4-[(1R,2R)-2-(4-fluorophenyl)-2-hydroxy-1-methyl-3-(1H-1,2,4-triazol-1-yl)propyl]-2-[4(1,1,2,2-tetrafluoroethoxy)phenyl]-3(2H,4H)-1,2,4-triazolone,

4-[(1R,2R)-2-(4-fluorophenyl)-2-hydroxy-1-methyl-3-(1H-1,2,4-triazol-1-yl)propyl]-2-[4-(2,2,3,3-tetrafluoropropoxy)phenyl]-3(2H,4H)-1,2,4-triazolone and the like.

Preferred examples of the compounds of (IV) include in addition to those mentioned for the compound (IV') as above:

2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone,

4-[(1R,2R)-2-(2,4-difluorophenyl)-2-hydroxy-1-methyl-3-(1H-1,2,4-triazol-1-yl)propyl]-2-[4-(2,2,3,3-tetrafluoropropoxy)phenyl]-3(2H,4H)-1,2,4-triazolone,

4-[(1R,2R)-2-(2,4-difluorophenyl)-2-hydroxy-1-methyl-3-(1H-1,2,4-triazol-1-yl)propyl]-2-[4-(1,1,2,2-tetrafluoroethoxy)phenyl]-3(2H,4H)-1,2,4-triazolone,

2-[(1R,2R)-2-(2,4-difluorophenyl)-2-hydroxy-1-methyl-3-(1H-1,2,4-triazol-1-yl)propyl]-5-methyl-4-[4-(1,1,2,2-tetrafluoroethoxy)phenyl]-3(2H,4H)-1,2,4-triazolone and the like.

The compound (IV) or salt thereof can be produced by reacting the compound (I) or salt thereof with the compound (III).

The reaction can be conducted by employing about 1 to 4 moles, more preferably about 1.5 to 2 mole of the compound (I) or salt thereof per 1 mole of the compound (III).

The reaction can be conducted in a solvent which do not impede reaction. Examples of such solvents are ketones such as acetone; sulfoxides such as dimethyl sulfoxide; ethers such as diethyl ether, tetrahydrofuran and dioxane; nitriles such as acetonitrile; aromatic hydrocarbons such as benzene, toluene and xylene; halogenated hydrocarbons such as dichloromethane, chloroform and 1,2-dichloroethane; esters such as ethyl acetate; amides such as dimethylformamide, acetamide, dimethylacetamide, 1-methyl-2-pyrrolidone and 1,3-dimethyl-2-imidazolidinone; and the like. Preferably, the solvents are sulfoxides such as dimethyl sulfoxide and amides such as dimethylformamide, acetamide, dimethylacetamide, 1-methyl-2-pyrrolidone and 1,3-dimethyl-2-imidazolidinone. More preferably, the solvents are amides such as dimethylformamide, acetamide, dimethylacetamide, 1-methyl-2-pyrrolidone and 1,3-dimethyl-2-imidazolidinone, among which dimethylformamide is most preferable. They may be used either singly or as a mixture thereof in a suitable mixing ratio.

The amount of the solvents used is usually about 25 times or less by weight to the compound (III), preferably about 5 to 25 times by weight, most preferably about 10 to 25 times by weight.

The reaction temperature ranges from about 50 to about 110 °C, more preferably from about 70 to about 100 °C.

The reaction time is not particularly limited but ranges usually about 20 to about 150 hours, preferably about 30 to about 100 hours.

Further, it is preferred that the above reaction is conducted in the presence of a base such as an alkali metal carbonate, alkali metal hydrogencarbonate, alkali metal hydroxide, alkali metal hydride, alkali metal organic carboxylate, alkali metal alcoholate, tetrabutylammonium fluoride and the like. Preferably, it is conducted in the presence of an alkali metal carbonate, alkali metal hydrogencarbonate or alkali metal hydroxide, more preferably alkali metal carbonate or alkali metal hydrogencarbonate, and most preferably alkali metal carbonate.

Examples of the alkali metal carbonates include lithium carbonate, sodium carbonate, cesium carbonate and potassium carbonate, among which potassium carbonate is preferable. Examples of the alkali metal hydrogencarbonates include lithium hydrogencarbonate, potassium hydrogencarbonate and sodium hydrogencarbonate. Examples of the alkali metal hydroxides include lithium hydroxide, potassium hydroxide and sodium hydroxide. Examples of the alkali metal hydrides include potassium hydride and sodium hydride. Examples of the alkali metal organic carboxylates include sodium acetate or the like. Examples of the alkali metal alcoholate include sodium methylate and potassium tert-butylate.

The amount of the base used is usually about 1 to about 10 equivalents, preferably about 1.5 to about 4 equivalents, to the compound of formula (I).

A method for adding the base is not especially limited. For example, the base may be added after adding the compound (I) or salt thereof and the compound (III) to the solvent, or the compound (I) or salt thereof and the compound (III) are added to a mixture prepared by adding the base to the solvent, or the compound (I) or salt thereof, the compound (III) and the alkali metal carbonate are added to the solvent in sequence. Further, the compound of (III) and the base may be added to the solvent, then the mixture is heated at about 60 to 100 °C for about 30 minutes to about 2 hours, followed by adding the compound (I) or salt thereof.

The compound (IV) or salt thereof can be also produced by reacting the compound (I) or salt thereof with the compound (II) in the presence of an alkali metal carbonate.

The reaction can be conducted by employing about 1 to 4 moles, preferably about 1.5 to 2 moles of the compound (I) per 1 mole of the compound (II).

Examples of the alkali metal carbonates used in the above reaction include lithium carbonate, sodium carbonate, cesium carbonate and potassium carbonate, among which potassium carbonate is preferable.

The amount of the alkali metal carbonate used is about 1 to 10 times by mole to the compound (I), preferably about 1.5 to 4 times by mole.

The reaction can be preferably conducted in a solvent. Examples of the solvents used for the present invention are those which do not impede the reaction, specifically ketones such as acetone; sulfoxides such

as dimethyl sulfoxide; ethers such as diethyl ether, tetrahydrofuran and dioxane; nitriles such as acetonitrile; aromatic hydrocarbons such as benzene, toluene and xylene; halogenated hydrocarbons such as dichloromethane, chloroform and 1,2-dichloroethane; esters such as ethyl acetate; amides such as dimethylformamide, acetamide, dimethylacetamide, 1-methyl-2-pyrrolidone and 1,3-dimethyl-2-imidazolidinone; and the like. Preferably, the solvents are sulfoxides such as dimethyl sulfoxide and amides such as dimethylformamide, acetamide, dimethylacetamide, 1-methyl-2-pyrrolidone and 1,3-dimethyl-2-imidazolidinone. More preferably, the solvents are amides such as dimethylformamide, acetamide, dimethylacetamide, 1-methyl-2-pyrrolidone and 1,3-dimethyl-2-imidazolidinone, among which dimethylformamide is most preferable. They may be used either singly or as a mixture thereof in a suitable mixing ratio.

The amount of the solvents used is usually about 25 times or less by weight to the compound (II) or salt thereof, preferably about 5 to 25 times by weight, most preferably about 10 to 25 times by weight.

A method for adding the above described alkali metal carbonate is not especially limitted. For example, the alkali metal carbonate may be added after adding the compound (I) and the compound (II) to the solvent, or the compound (I) and the compound (II) are added to a mixture prepared by adding the alkali metal carbonate to the solvent, or the compound (I), the compound (II) and the alkali metal carbonate are added to the solvent in sequence.

The reaction temperature is preferably about 50 to 110 °C, more preferably about 70 to 100 °C.

The reaction time is preferable about 20 to 150 hours, more preferably about 30 to 100 hours.

The compound of formula (IV) or (IV') can be also used as a salt and examples of such salts are pharmacologically-acceptable salts such as inorganic acid salts (e.g., hydrochloride, hydrobromide, sulfate, nitrate and phosphate) or organic acid salts (e.g., acetate, tartrate, citrate, fumarate, maleate, p-toluenesulfonate and methanesulfonate).

The compound of formula (IV) or (IV') or salt thereof can be isolated from the reaction mixture by known isolation and purification procedure such as extraction, concentration, neutralization, filtration, recrystallization, chromatography.

The salt of the compound of formula (IV) or (IV') can also be manufactured by a method such as by adding the above-mentioned inorganic or organic acid to the compound of the formula (IV).

The compound of formula (I) or salt thereof, which is a starting material of the present invention, can be produced by a known method, for example, a method disclosed in European Laid Open Patent Publication 567982 and the like. The compound (IX) or salt thereof in which Y and Z of the compound (I) are CH and N, respectively is produced by a method as given in the following scheme.

Examples of the salt of the compound (I) include alkali metal salts which are formed of a metal such as lithium, sodium, potassium, cesium and the like.

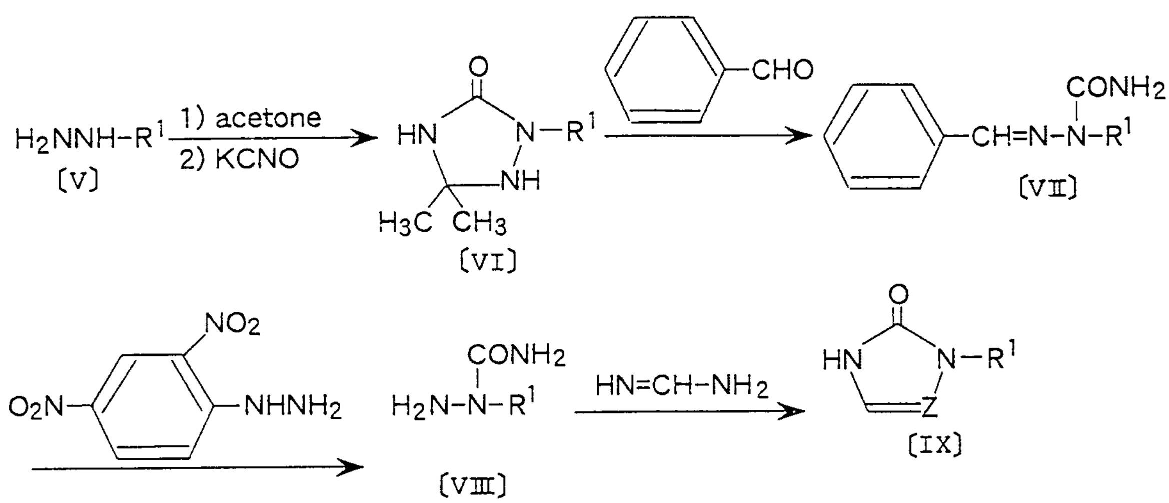

[V] [VI] [VII] [VIII] [IX]

wherein the symbols have the same meanings as above,

The compounds of formulae (II) and (III) or salts thereof, which are starting materials of the present invention, can be produced by a known method, for example, a method disclosed in Japanese Laid Open Patent Publications Hei 4 (1992)-74168, Hei 5(1993)-230038 and European Laid Open Patent Publication 548553 and the like. Particularly, the compound (III) or salt thereof in which $R^2$ of the compound (XI) is p-tolyl group is produced by a method as given in the following scheme.

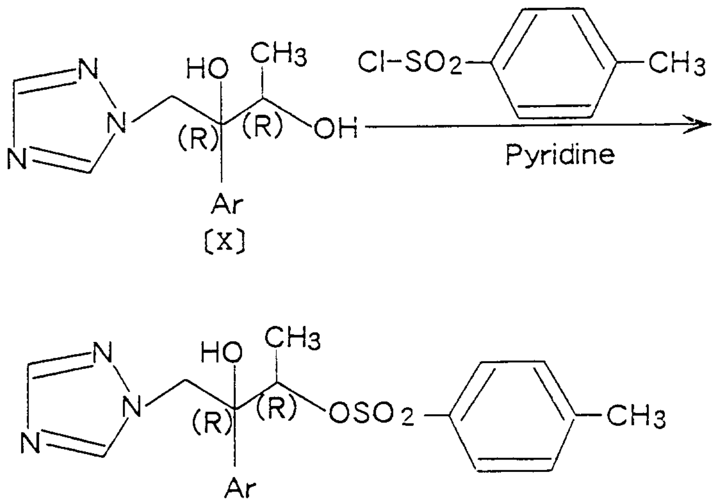


[X]

[XI]

wherein the symbols have the same meanings as above.

The compound of formula (X) or salt thereof, which is a starting material of the present invention, can be produced by a known method, for example, a method disclosed in Japanese Laid Open Patent Publications Hei 5(1993)-230038 and European Laid Open Patent Publications 548553 and 567982 and the like.

The salt used for the compounds (II) and (III) which are starting materials is the same as the salt used for the compound (IV).

The compounds (I), (II) and (III) or salts thereof which are obtained by the method described above, can be isolated from the reaction mixture by known isolation and purification procedure such as extraction, concentration, neutralization, filtration, recrystallization, chromatography.

The compound (IV) or (IV') which is produced by the method of the present invention possesses a low toxicity, high antifungal activity and broad antifungal spectrum against various fungi (e.g., ***Candida, Aspergillus*** and ***Cryptococous***). Accordingly it can be used for prevention or treatment of fungus-infectious diseases (e.g., candidiasis, aspergillosis and cryptococosis) to mammals (e.g., human, farm animal, poultry and the like). In addition, the compound (IV) or salt thereof can be used as an agricultural antifungal agent.

The compound of formula (IV') or salt thereof can be safely administered, either orally or parenterally, to human beings in the form of pharmaceutical compositions such as oral administration preparations (e.g., powder, granules, tablets or capsules), parental preparations [e.g., injections, external preparations (e.g., nasal or dermatological ones), suppositories (e.g., rectal or vaginal ones) and so on] in per se or by mixing with suitable pharmacologically acceptable carriers, fillers or diluents.

Those preparations can be manufactured by the methods which are known per se and commonly used in the manufacture of pharmaceutical preparations.

For example, the compound of formula (IV') or salt thereof of the present invention can be made into injections such as aqueous injection together with dispersing agent (e.g., Tween 80 [Atlas Powder, U. S. A.), HCO 60 [Nikko Chemicals, Japan], carboxymethylcellulose and sodium alginate), preservative (e.g., methylparaben, propylparaben, benzyl alcohol or chlorobutanol), isotonic agent (e.g., sodium chloride, glycerin, sorbitol or glucose) and the like, or as oily injection by dissolving, suspending or emulsifying in plant oil (e.g., olive oil, sesame oil, peanut oil, cotton seed oil or corn oil), propylene glycol and the like.

In the manufacture of preparations for oral administraion, the compound of formula (IV')or salt thereof of the present invention is molded with pressure together, for example, with fillers (e.g., lactose, sugar or starch), disintegrating agents (e.g., starch or calcium carbonate), binders (e.g., starch, arabic gum, carboxymethylcellulose, polyvinylpyrrolidone or hydroxypropylcellulose), lubricants (e.g., talc, magnesium stearate or polyethyleneglycol 6000) and the like followed, if necessary, by coating in accordance with a known method per se with an object of taste-masking or of providing the preparation with enteric or sustained release property. Examples of the coating agents are, for example, hydroxypropylmethylcellulose, ethylcellulose, hydroxymethylcellulose, hydroxypropylcellulose, polyoxyethylene glycol, Tween 80, Pluronic

F68, cellulose acetate phthalate, hydroxypropylmethylcellulose phthalate, hydroxymethylcellulose acetate succinate, Eudoragit (Rohm, West Germany; a copolymer of methacrylic acid with acrylic acid) and pigments such as titanium oxide and red iron oxide.

In the case of preparation for external use, the compound of formula (IV')or salt thereof of the present invention can be, for example, made into solid, semisolid or liquid preparation by a known method per se. For example, in the case of solid preparation, the compound of formula (IV')or salt thereof is used as it is or mixed with fillers (e.g., glucose, mannitol, starch or microcrystalline cellulose), thickeners (e.g., natural gums, cellulose derivatives or acrylic acid polymers), etc. to give powdered composition. In the case of liquid preparation, the procedures are nearly the same as those in the case of injections to give oily or aqueous suspension. In the case of semisolid preparation, aqueous or oily gel or ointment is preferred. All of those can be added with pH adjusting agents (e.g., carbonic acid, phosphoric acid, citric acid, hydrochloric acid or sodium hydroxide), antiseptics (e.g., p-hydroxybenzoates, chlorobutanol or benzalkonium chloride) and the like. For example, it can be used for sterilization or disinfection of skin or mucous membrane as an ointment preparation containing about 0.1 to 100 mg of it per gram using vaselin or lanoline as a base material.

In the case of suppositories, the compound of the present invention (IV')or salt thereof can be made, by a known method per se, into oily or aqueous suppositories in solid, semisolid or liquid. Examples of the oily base materials used therefor are higher fatty acid glycerides (e.g., cacao butter, Witepsols [Dynamite-Nobel], medium fatty acids (e.g., Migriol [Dynamite-Nobel]), plant oil (e.g., sesame oil, soybean oil or cotton seed oil) and the like. Examples of the aqueous base materials are polyethylene glycol and propylene glycol while examples of aqueous gel base materials are natural gums, cellulose derivatives, vinyl polymers and acrylic acid polymers.

Dose may vary depending upon the state of infection and the administering route and, in giving to adult patient (body weight: 50 kg) for therapy of candidosis by oral route, it is about 0.01 to 100 mg/kg/day, preferably about 0.1 to 50 mg/kg/day and, more preferably, about 0.1 to 20 mg/kg/day.

When it is used as an antifugal agent for agricultural purposes, the compound of formula (IV') or salt thereof is dissolved or dispersed in a suitable liquid carrier (e.g., solvent) or mixed or adsorbed with a suitable solid carrier (e.g., diluent or filler) followed, if necessary, by adding emulsifier, suspending agent, spreader, penetrating agent, moisturizing agent, thickening agent, stabilizer, etc. to afford the preparation such as emulsion, hydrated agent, powder, granules. Such preparations can be prepared by known method per se. The amount of the compound of the formula (IV') or salt thereof used is, for example in the case of rice blast disease, about 25 to 150 g, preferably about 40 to 80 g, per are of irrigated rice field.

Examples of the liquid carrier used are water, alcohols (e.g., methyl alcohol, ethyl alcohol, n-propyl alcohol, isopropyl alcohol or ethylene glycol), ethers (e.g., dioxane or tetrahydrofuran), aliphatic hydrocarbons (e.g., kerosene, lamp oil or fuel oil), aromatic hydrocarbons (e.g., benzene or toluene), halogenated hydrocarbons (e.g., methylene chloride or chloroform), acid amides (e.g., dimethylformamide or dimethylacetamide), esters (e.g., ethyl acetate or butyl acetate), nitriles (e.g., acetonitrile or propionitrile) and the like. They may be used either singly or as a mixture thereof in a suitable mixing ratio.

Examples of the solid carrier are plant powder (e.g., soybean powder, tobacco powder or wheat flour), mineral powder (e.g., kaolin or bentonite), alumina, sulfur powder, activated charcoal, etc. They may be used either singly or as a mixture thereof by mixing in a suitable ratio.

The present invention is further described by way of the following Reference Examples and Working Examples.

$^1$H-NMR spectra were measured by a spectrometer of Varian Gemini 200 type (200MHz) using tetramethylsilane as an internal standard. All $\delta$ value are given by ppm. In the mixing solvents, the figures given in ( ) are the mixing ratio of each of the solvents by volume. Unless otherwise specified, the symbol % means that by weight. In the silica gel chromatography, the ratio of the solvents is a ratio of the mixed solvents by volume.

The symbols used in the examples have the following meanings.

s: singlet; d: doublet; t: triplet; q: quartet; dd: double doublet; tt: triple triplet; m: multiplet; quintet:quintet; septet:septet; br: broad; J: coupling constant.

Reference Example 1

4-(1,1,2,2-Tetrafluoroethoxy)aniline (29.54 g) was mixed with 141 ml of concentrated hydrochloric acid, to which an aqueous solution of 10.24 g of sodium nitrite in 50 ml of water was added dropwise with stirring at -10 °C. After the mixture was stirred with ice cooling for 30 minutes, 128.6 g of stannous chloride in 130 ml of concentrated hydrochloric acid was added thereto with ice cooling, and the resulting mixture was

stirred for 30 minutes. To the reaction mixture was added a solution of 200 g of sodium hydroxide in 300 ml of water to adjust pH in the range of 12 to 14. The insoluble substance was removed by filtration on Celite and the filtrate was extracted with dichloromethane (300 ml X 3). The extracts were combined, washed with 300 ml of water and dried over anhydrous magnesium sulfate. The solvent was distilled off under reduced pressure to give 27.2 g of 4-(1,1,2,2-tetrafluoroethoxy)phenyl hydrazine as an oily substance. This product was dissolved in 50 ml of ethanol, to which 16 ml of acetone was added, and the resulting solution was stirred at room temperature for 30 minutes. After the solvent was distilled off under reduced pressure, the residue was dissolved in 75 % acetic acid aqueous solution (200 ml), to which 9.86 g of potassium cyanate was added, and the resulting mixture was stirred at 60 °C for an hour. After cooling, the reaction solution was diluted with 200 ml of water and neutralized with ammonia water with ice cooling to adjust pH 7.0. The precipitated crystals were collected by filtration, washed with 100 ml of water and 100 ml of hexane successively, and dried under reduced pressure to give 24.1 g of 3,3-dimethyl-1-[4-(1,1,2,2-tetrafluoroethoxy)phenyl]-1,2,4-triazolidine-5-on as a pale brown crystalline powder.

$^1$H-NMR ($CDCl_3$) δ: 1.49 (6H,s), 4.55 (1H,s), 5.29 (1H,s), 5.89 (1H,tt,J=53Hz,J=2.8Hz), 7.16 (2H,d,J=8.8Hz), 7.72 (2H,d,J=8.8Hz).

The brown crystalline powder obtained above (24.1 g) was suspended in 160 ml of water, to which 13.3 ml of concentrated hydrochloric acid and 12.65 g of benzaldehyde were added. The reaction solution was stirred at 80 °C for 2.5 hours and cooled. The precipitated crystals were collected by filtration, washed with 100 ml of water and then with 200 ml of hexane, and dried under reduced pressure to give 25.6 g of benzaldehyde 2-[4-(1,1,2,2-tetrafluoroethoxy)phenyl]-semicarbazone as a pale brown crystalline powder.

$^1$H-NMR ($CDCl_3$) δ: 5.96 (1H,tt,J=53Hz,J=2.8Hz), 7.11-7.72(10H,m)

This crystalline powder (15 g) was dissolved in 200 ml of ethanol, to which 13.65 g of 2,4-dinitrophenyl hydrazine monohydrate and 0.5 ml of concentrated hydrochloric acid were added. The mixture was stirred at 70 °C for 17 hours and cooled. The insoluble substance was removed by filtration and the filtrate was concentrated under reduced pressure. The residue was purified by silica gel chromatography (eluate; ethyl acetate:methanol = 5:1) to give 8.68 g of 2-[4-(1,1,2,2-tetrafluoroethoxy)phenyl]semicarbazide as colorless prisms.

$^1$H-NMR ($CDCl_3$) δ: 4.31 (2H,s), 5.64 (2H,s), 5.89 (1H,tt,J=53Hz,J=2.8Hz), 7.20 (2H,d,J=9Hz), 7.49 (2H,d,J=9Hz).

The above product (3.15 g) and 6.11 g of formamidine acetate were dissolved in 25 ml of dimethylformamide, and the solution was stirred at room temperature for an hour. To the reaction solution was added 3.52 g of acetic acid, and the resulting mixture was stirred at 80 °C for 6 hours. After cooling, the mixture was poured into 50 ml of ice water and extracted with ethyl acetate (25 ml X 3). The extracts were combined, washed with water (25 ml X 3) and dried over anhydrous magnesium sulfate. After distilling off the solvent under reduced pressure, the residue was purified by silica gel chromatography (eluate; ethyl acetate:hexane = 1:1 to 3:2) and crystallized from diisopropyl ether to give 2.16 g of 2-[4-(1,1,2,2-tetrafluoroethoxy)phenyl]-3(2H,4H)-1,2,4-triazolone as a colorless crystalline powder.

mp 192-193 °C

| Elemental analysis for $C_{10}H_7F_4N_3O_2$ | | | |
|---|---|---|---|
| Calcd: | C 43.33, | H 2.55, | N 15.16 |
| Found: | C 43.26, | H 2.54, | N 15.13 |

$^1$H-NMR ($d_6$-DMSO) δ: 6.81 (1H,tt,J=49.8Hz,J=1.8Hz), 7.37 (2H,d,J=8.4Hz), 8.00 (2H,d,J=8.4Hz), 8.15 (1H,s).

Reference Example 2

In the same manner as that described in Reference Example 1, 2-[4-(2,2,3,3-tetrafluoropropoxy)phenyl]-3(2H,4H)-1,2,4-triazolone was obtained as a colorless crystalline powder , starting from 4-(2,2,3,3-tetrafluoropropoxy)aniline.

mp 186-189 °C

| Elemental analysis for $C_{11}H_9F_4N_3O_2$ | | | |
|---|---|---|---|
| Calcd: | C 45.37, | H 3.12, | N 14.43 |
| Found: | C 45.62, | H 3.18, | N 14.39 |

$^1$H-NMR ($d_6$-DMSO) δ: 4.60 (2H,t,J = 12.8Hz), 6.68 (1H,tt,J = 52.2Hz,J = 5.6Hz), 7.13 (2H,d,J = 9Hz), 7.81 (2H,d,J = 9Hz), 8.08 (1H,s).

Reference Example 3

60% of sodium hydride in oil (3.52 g) was dispersed in 60 ml of dimethyl sulfoxide, to which trimethylsulfoxonium iodide (21.14 g) was added portionwise under a nitrogen atmosphere with ice cooling. The reaction solution was stirred at room temperature for an hour, to which dichloromethane (484 ml) was added and cooled with ice again. A solution of 20 g (98.7% ee) of (2R)-2',4'-difluoro-2-(3,4,5,6-tetrahydro-2H-pyran-2-yloxy)propiophenone in 67 ml of dichloromethane was added over a period of 10 minutes and the resulting mixture was stirred at room temperature for 2 hours. The reaction mixture was diluted with 500 ml of cold water to separate the dichloromethane layer. The dichloromethane layer was washed with water (100 ml x 2) and saturated aqueous solution of sodium chloride (100 ml) successively, and dried over anhydrous magnesium sulfate. The solvent was removed under reduced pressure to give 2-(2,4-difluorophenyl)-2-[(1R)-1-(3,4,5,6-tetrahydro-2H-pyran-2-yloxy)ethyl]oxirane (21.4 g, containing a mineral oil) as a pale yellow oil.

The above pale yellow oil (21.4 g), 1H-1,2,4-triazole (10.18 g) and potassium carbonate (30.6 g) was added to 180 ml of di-methylformamide, and the resulting mixture was heated with stirring at 80 °C for 12 hours. After cooling, the reaction mixture was poured into 400 ml of ice water and extracted with ethyl acetate (250 ml x 2). The ethyl acetate layer was washed with water (250 ml x 2) and saturated aqueous solution of sodium chloride (250 ml) successively, and dried over anhydrous magnesium sulfate. The solvent was removed under reduced pressure, and the residue was purified by silica gel chromatography (eluate; ethyl acetate:hexane = 2:1 to ethyl acetate: acetone = 10:1) to give (3R)-2-(2,4-difluorophenyl)-3-(3,4,5,6-tetrahydro-2H-pyran-2-yloxy)-1-(1H-1,2,4-triazol-1-yl)-2-butanol (19.8 g) as a pale yellow oily substance.

This product (19.8 g) was dissolved in 170 ml of methanol, to which 10.83 g of p-toluenesulfonic acid monohydrate was added and the resulting solution was stirred at room temperature for an hour. The reaction solution was neutralized with potassium carbonate solution ($K_2CO_3$ 3.89 g/water 30 ml) and concentrated at the temperature under 35 °C under reduced pressure. The residue was diluted with 20 ml of water and extracted with ethyl acetate (100 ml x 3). The ethyl acetate layer was washed with saturated aqueous solution of sodium chloride (50 ml) and dried over anhydrous magnesium sulfate. The solvent was removed off under reduced pressure to give a diol compound as a white solid (12.34 g).

The diol compound (12.1 g) was added in 80 ml of ethyl acetate and dissolved by heating at 80 °C. After allowing to stand at room temperature for 12 hours, the precipitated crystals were removed by filtration and the crystals were washed with ethyl acetate (5 ml). The filtrate and washing were combined and distilled off under reduced pressure. Diisopropyl ether (60 ml) was added to the residue and the mixture was stirred with ice cooling for an hour. The precipitated crystals were collected by filtration, washed with diisopropyl ether (5 ml) and dried to give (2R,3R)-2-(2,4-difluorophenyl)-1-(1H-1,2,4-triazol-1-yl)-2,3-butandiol (9.0 g).

Reference Example 4

(2R,3R)-2-(2,4-Difluorophenyl)-1-(1H-1,2,4-triazol-1-yl)-2,3-butandiol (2g: synthesized by the method disclosed in Japanese Laid Open Publications Hei 4-74168 and 5-230038 or by the method described in Reference Example 3) was dissolved in 10 ml of pyridine, to which 1.58 g of p-toluenesulfonyl chloride was added with ice cooling. The reaction solution was stirred with ice cooling for 30 minutes and then at room temperature for 20 hours. To the reaction solution was added 2 ml of water with ice cooling and the mixture was stirred with ice cooling for 15 minutes and then at room temperature for 15 minutes. The reaction mixture was poured into 100 ml of ice water and extracted with ethyl acetate (50 ml X 3). The extracts were combined and washed with 0.5N-hydrochloric acid (50 ml X 4), water (50 ml), 0.5N-sodium hydroxide (50 ml) and water (50 ml) successively. The ethyl acetate layer was dried over anhydrous magnesium sulfate and the solvent was distilled off under reduced pressure. The residue was purified with silica gel chromatography (eluate ; dichloromethane:ethyl acetate = 2:1) to give (2R,3R)-2-(2,4-difluorophenyl)-3-(p-toluenesulfonyloxy)-1-(1H-1,2,4-triazol-1-yl)-2-butanol(2.3 g, yield: 73%) as a colorless crystalline powder.

$^1$H-NMR ($CDCl_3$) δ: 1.04 (3H,d,J=6.6Hz), 2.47(3H,s), 4.64 (1H,d,J=14Hz), 4.82 (1H,s), 4.91 (1H,d,J=14Hz), 5.15(1H,q,J=6.6Hz), 6.68-6.78(2H,m), 7.27-7.45(1H,m), 7.38(2H,d,J=8Hz), 7.76(1H,s) 7.83-(1H,s), 7.85(2H,d,J=8Hz).

Reference Example 5

(2R,3R)-2-(2,4-Difluorophenyl)-1-(1H-1,2,4-triazol-1-yl)-2,3-butanediol (2g) was dispersed in 20 ml of dichloromethane. To the mixture was added with ice cooling 1.14 ml of triethylamine and 0.63 ml of methanesulfonyl chloride. The reaction mixture was stirred at room temperature for 45 minutes and then washed with 20 ml of water and 20 ml of saturated aqueous solution of sodium chloride successively. The dichloromethane layer was dried over anhydrous magnesium sulfate, and the solvent was distilled off under reduced pressure. The residue was purified by silica gel chromatography (eluate ; ethyl acetate:dichloroethane = 4:1) and crystallized from dichloromethane-diisopropyl ether to give (2R,3R)-2-(2,4-difluorophenyl)-3-methanesulfonyloxy-1-(1H-1,2,4-triazol-1-yl)-2-butanol (2.1 g, yield: 81%) as a colorless crystalline powder.

mp 115-116 °C

$[\alpha]^{20}_D$-71.9 ° (c=1, in methanol)

| Elemental analysis for $C_{13}H_{15}F_2N_3O_4S$ | | | |
|---|---|---|---|
| Calcd: | C 44.95, | H 4.35, | N 12.10 |
| Found: | C 44.87, | H 4.29, | N 12.19 |

$^1$H-NMR ($CDCl_3$) δ: 1.29 (3H,d,J=6.6Hz), 3.11 (3H,s), 4.68 (1H,d,J=14.2Hz), 5.03 (1H,d,J=14.2Hz), 5.07 (1H,s), 5.28 (1H,q,J=6.6Hz), 6.71-6.84(1H,m), 7.40-7.53(2H,m), 7.79(1H,s),7.87(1H,s).

Example 1

Preparation of 2-[(1R,2R)-2-(2,4-difluorophenyl)-2-hydroxy-1-methyl-3-(1H-1,2,4-triazol-1-yl)propyl]-4-(4-methylthiophenyl)-3(2H,4H)-1,2,4-triazolone.

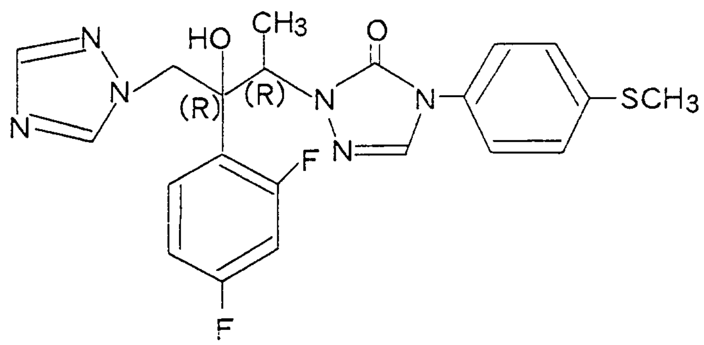

(2R,3S)-2-(2,4-difluorophenyl)-3-methyl-2-[(1H-1,2,4-triazol-1-yl)methyl]oxirane (3.29 g), 4-(4-methylthiophenyl)-3(2H,4H)-1,2,4-triazolone (4.0 g) and potassium carbonate (powder: 4.5 g) were added to 30 ml of N,N-dimethylformamide, and the resulting mixture was heated at 80 °C for 24 hours with stirring and then at 100 °C for 4 hours. The reaction mixture was cooled, diluted with 100 ml of ethyl acetate and ice water (100 ml) to separate the ethyl acetate layer. The aqueous layer was extracted with ethyl acetate (50 ml X 2). The ethyl acetate layers were combined and washed with 0.5N-sodium hydroxide (50 ml X 3), 1N-hydrochloric acid (50 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; ethyl acetate:hexane = 3:2 to ethyl acetate) and crystallized from diisopropyl ether-hexane (2:1) to give the title compound (2.86 g, yield: 48 %) as colorless crystals.

mp 151-153 °C

Example 2

Preparation of 4-[(1R,2R)-2-(2,4-difluorophenyl)-2-hydroxy-1-methyl-3-(1H-1,2,4-triazol-1-yl)propyl]-2-[4-(2,2,3,3-tetrafluoropropoxy)phenyl]-3(2H,4H)-1,2,4-triazolone.

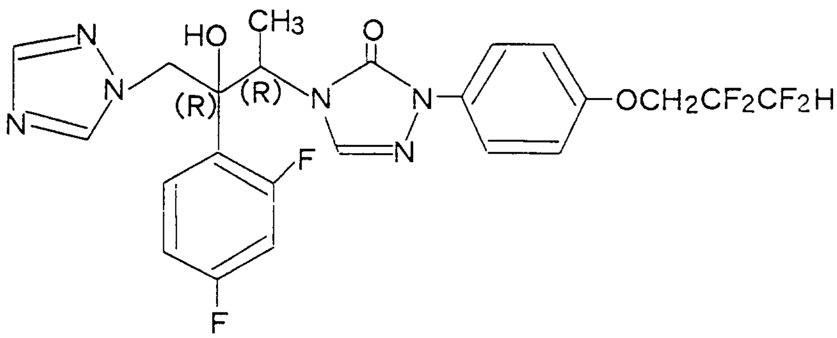

(2R,3S)-2-(2,4-Difluorophenyl)-3-methyl-2-[(1H-1,2,4-triazol-1-yl)methyl]oxirane (3.6 g), 2-[4-(2,2,3,3-tetrafluoropropoxy)phenyl)-3(2H,4H)-1,2,4-triazolone (8.33 g) and potassium carbonate (powder: 9.88 g) were added to 60 ml of N,N-dimethylformamide, and the resulting mixture was heated at 80 °C for 64 hours with stirring. The reaction mixture was cooled and diluted with 200 ml of ethyl acetate. Ice water (200 ml) was added to separate the ethyl acetate layer. The aqueous layer was extracted with ethyl acetate (100 ml X 2). The ethyl acetate layers were combined and washed with 0.5N-sodium hydroxide (100 ml X 3), 1N-hydrochloric acid (100 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; ethyl acetate:hexane = 1:1) and crystallized from ethyl acetate-hexane (1:5) to give the title compound (5.2 g, yield: 67 %) as colorless crystals.

The crystals were recrystallized from a mixture of ethanol and water to give the colorless crystals.

mp 162-163 °C

| Elemental analysis for $C_{23}H_{20}F_6N_6O_3$ | | | |
|---|---|---|---|
| Calcd: | C 50.93, | H 3.72, | N 15.49 |
| Found: | C 51.03, | H 3.71, | N 15.71 |

$^1$H-NMR ($CDCl_3$) δ: 1.23 (3H,d,J = 7Hz), 4.14 (1H,d,J = 14.4Hz), 4.49 (2H,t,J = 13Hz), 4.94 (1H,dq,J = 7Hz,1.6Hz), 5.08 (1H,d,J = 14.4Hz), 5.54 (1H,d,J = 1.6Hz), 6.10 (1H,tt,J = 52Hz,5.4Hz), 6.71-6.89 (2H,m), 7.03 (2H,d,J = 9.2Hz), 7.34-7.51 (1H,m), 7.77 (1H,s), 7.80 (1H,s), 7.93 (1H,s), 7.95 (2H,d,J = 9.2Hz).

Example 3

Preparation of 4-[(1R,2R)-2-(2,4-difluorophenyl)-2-hydroxy-1-methyl-3-(1H-1,2,4-triazol-1-yl)propyl]-2-[4-(1,1,2,2-tetrafluoroethoxy)phenyl]-3(2H,4H)-1,2,4-triazolone.

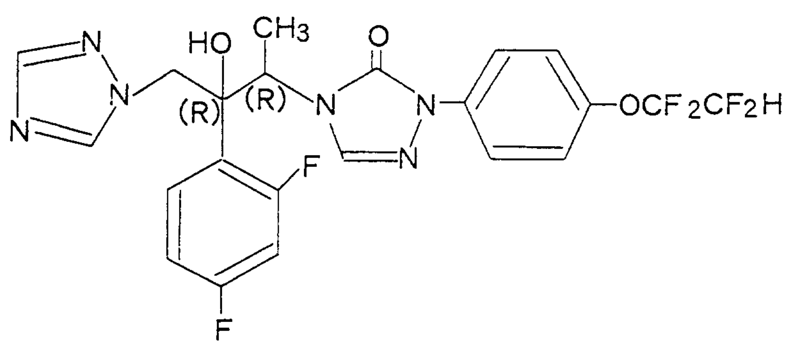

(2R,3S)-2-(2,4-Difluorophenyl)-3-methyl-2-[(1H-1,2,4-triazol-1-yl)methyl]oxirane (5.49 g), 2-[4-(1,1,2,2-tetrafluoroethoxy)phenyl)-3(2H,4H)-1,2,4-triazolone (12.11 g) and potassium carbonate (powder: 15.14 g) were added to 100 ml of N,N-dimethylformamide, and the resulting mixture was heated at 80 °C for 56 hours with stirring. The reaction mixture was cooled and diluted with 300 ml of ethyl acetate. Ice water (300 ml) was added to separate the ethyl acetate layer. The aqueous layer was extracted with ethyl acetate (100 ml x 2). The ethyl acetate layers were combined and washed with 0.5N-sodium hydroxide (100 ml x 3), 1N-hydrochloric acid (100 ml) and saturated aqueous solution of sodium chloride (100 ml) successively. The ethyl acetate layer was dried over anhydrous magnesium sulfate and concentrated under reduced pressure. The residue was purified by silica gel chromatography (eluate ; ethyl acetate:hexane = 1:1) and crystallized from ethyl acetate-hexane (1:5) to give the title compound (7.1 g, yield: 62 %) as colorless crystals.

The crystals were recrystallized from a mixture of ethanol and water to give the colorless crystals.

mp 155-156 °C

| Elemental analysis for $C_{22}H_{18}F_6N_6O_3$ | | | |
|---|---|---|---|
| Calcd: | C 50.01, | H 3.43, | N 15.90 |
| Found: | C 50.15, | H 3.35, | N 15.93 |

$^1$H-NMR ($CDCl_3$) δ: 1.24 (3H,d,J=7.2Hz), 4.13 (1H,d,J=14.2Hz), 4.95 (1H,dq,J=7.2Hz,1.6Hz), 5.08 (1H,d,J=14.2Hz), 5.55 (1H,d,J=1.6Hz), 5.93 (1H,tt,J=53Hz,2.8Hz), 6.71-6.90 (2H,m), 7.30 (2H,d,J=9.2Hz), 7.34-7.51 (1H,m), 7.77 (1H,s), 7.80 (1H,s), 7.96 (1H,s), 8.06 (2H,d,J=9.2Hz).

Example 4

Preparation of 4-[(1R,2R)-2-(2,4-difluorophenyl)-2-hydroxy-1-methyl-3-(1H-1,2,4-triazol-1-yl)propyl]-2-(4-trifluoromethoxyphenyl)-3(2H,4H)-1,2,4-triazolone.

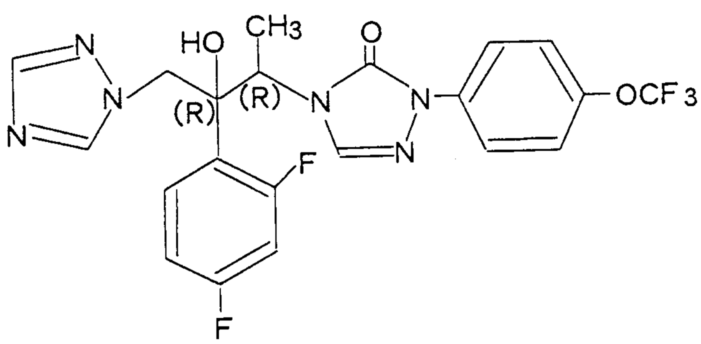

(2R,3S)-2-(2,4-Difluorophenyl)-3-methyl-2-[(1H-1,2,4-triazol-1-yl)methyl]oxirane (5.04 g), 2-(4-trifluoromethoxyphenyl)-3(2H,4H)-1,2,4-triazolone (9.84 g) and potassium carbonate (powder: 13.8 g) were added to 80 ml of N,N-dimethylformamide, and the resulting mixture was heated at 70 °C for 117 hours with stirring. The reaction mixture was cooled and diluted with 250 ml of ethyl acetate. Ice water (250 ml) was added to separate the ethyl acetate layer. The aqueous layer was extracted with ethyl acetate (100 ml x 2). The ethyl acetate layers were combined and washed with 0.5 N-sodium hydroxide (100 ml x 3), 1N-hydrochloric acid (100 ml) and saturated aqueous solution of sodium chloride (100 ml) successively. The ethyl acetate layer was dried over anhydrous magnesium sulfate and concentrated under reduced pressure. The residue was purified by silica gel chromatography (eluate ; ethyl acetate:hexane = 3:2) and crystallized from diisopropyl ether to give the title compound (5.64 g, yield: 57 %) as colorless crystals.

The crystals were dissolved in ethanol and diluted with water. The title compound precipitated as colorless crystals was collected by filtration.

| Elemental analysis for $C_{21}H_{17}F_5N_6O_3$ | | | |
|---|---|---|---|
| Calcd: | C 50.81, | H 3.45, | N 16.93 |
| Found: | C 50.66, | H 3.36, | N 17.02 |

$^1$H-NMR ($CDCl_3$) δ: 1.24 (3H,d,J=7.2Hz), 4.14 (1H,d,J=14.4Hz), 4.96 (1H,dq,J=7.2Hz,1.6Hz), 5.08 (1H,d,J=14.4Hz), 5.55 (1H,d,J=1.6Hz), 6.76-6.89 (2H,m), 7.31 (2H,d,J=9.2Hz), 7.48-7.52 (1H,m), 7.78 (1H,s), 7.80 (1H,s), 7.96 (1H,s), 8.08 (2H,d,J=9.2Hz).

Example 5

Preparation of 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

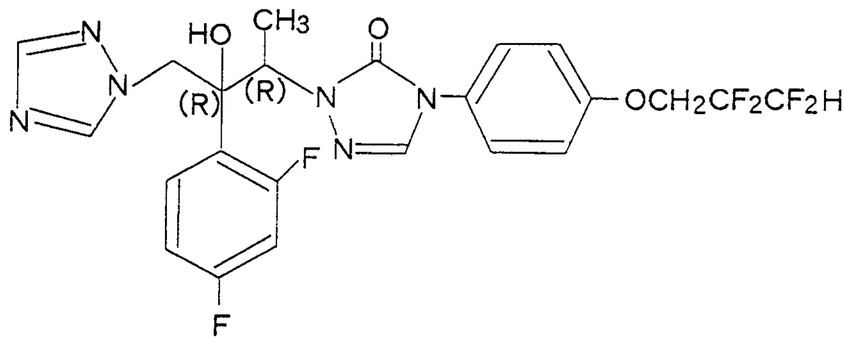

(2R,3S)-2-(2,4-Difluorophenyl)-3-methyl-2-[(1H-1,2,4-triazol-1-yl)methyl]oxirane (5.0 g), 4-[4-(2,2,3,3-tetrafluoropropoxy)phenyl]-3(2H,4H)-1,2,4-triazolone (8.4 g) and potassium carbonate (powder: 13.8 g) were added to 100 ml of N,N-dimethylformamide, and the resulting mixture was stirred at 80 °C for 27 hours and then at 100 °C for 4 hours. The reaction mixture was cooled and diluted with 800 ml of ethyl acetate. The mixture was washed with water (200 ml), 1N-sodium hydroxide water (200 ml x 3), 1N-hydrochloric acid (200 ml x 2) and saturated aqueous solution of sodium chloride (100 ml) successively. The ethyl acetate layer was dried over anhydrous magnesium sulfate and concentrated under reduced pressure. Diisopropyl ether (20 ml) was added to the residue and the mixture was cooled in an ice bath to give a pale brown powder (8.5 g). The powder was recrystallized from ethyl acetate-diisopropyl ether (1:3, 40 ml) to give the title compound (6.88 g) as colorless crystals. The mother liquors were combined and concentrated. The residue was purified by silica gel chromatography (eluate ; hexane:ethyl acetate = 2:3 to 1:5) and crystallized from ethyl acetate-diisopropyl ether (1:8 to 18 ml) to give the title compound (1.12 g).

Total yield: 8.0 g (yield: 74 %)

The title compound was recrystallized from a mixture of ethanol and water to give the colorless crystals.

mp 154-155 °C

| Elemental analysis for $C_{23}H_{20}F_6N_6O_3$ | | | |
|---|---|---|---|
| Calcd: | C 50.93, | H 3.72, | N 15.49 |
| Found: | C 50.85, | H 3.59, | N 15.54 |

$^1$H-NMR ($CDCl_3$) δ: 1.30 (3H,d,J=7Hz), 4.36 (1H,d,J=15Hz), 4.40 (2H,tt,J=11.8Hz,1.4Hz), 5.01 (1H,d,J=15Hz), 5.09 (1H,q,J=7Hz), 5.47 (1H,s), 6.07 (1H,tt,J=53Hz,4.8Hz), 6.75-6.88 (2H,m), 7.07 (2H,dt,J=9Hz, J=2.2Hz), 7.53 (2H,dt,J=9Hz,J=2.2Hz), 7.48-7.64 (1H,m), 7.69 (1H,s), 7.75 (1H,s), 7.95 (1H,s).

Example 6

Preparation of 2-[(1R,2R)-2-(2,4-difluorophenyl)-2-hydroxy-1-methyl-3-(1H-1,2,4-triazol-1-yl)propyl]-5-methyl-4-[4-(1,1,2,2-tetrafluoroethoxy)phenyl]-3(2H,4H)-1,2,4-triazolone.

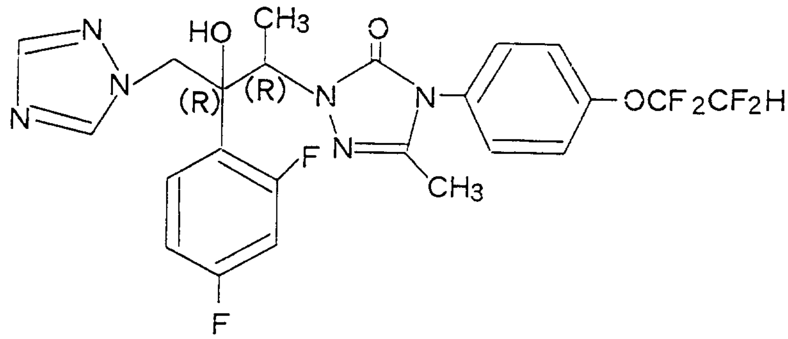

(2R,3S)-2-(2,4-Difluorophenyl)-3-methyl-2-[(1H-1,2,4-triazol-1-yl)methyl]oxirane (502 mg), 5-methyl-4-[4-(1,1,2,2-tetrafluoroethoxy)phenyl]-3(2H,4H)-1,2,4-triazolone (873 mg) and potassium carbonate (powder: 1.38 g) were added to 10 ml of N,N-dimethyl formamide, and the resulting mixture was stirred at 80 °C for 26 hours and then at 100 °C for 8 hours. The reaction mixture was cooled, diluted with 80 ml of ethyl acetate and washed with water (20 ml), 1N-sodium hydroxide water (20 ml), 1N-hydrochloric acid (20 ml) and saturated aqueous solution of sodium chloride (10 ml) successively. The ethyl acetate layer was dried over anhydrous magnesium sulfate and concentrated under reduced pressure. The residue was purified by silica gel chromatography (eluate ; hexane:ethyl acetate = 1:2 to 1:4) to give the title compound (0.78 g, yield: 72 %) as a colorless powder.

| Elemental analysis for $C_{23}H_{20}F_6N_6O_3$ | | | |
|---|---|---|---|
| Calcd:<br>Found: | C 50.93,<br>C 50.53, | H 3.72,<br>H 3.77, | N 15.49<br>N 15.26 |

$^1$H-NMR ($CDCl_3$) δ: 1.26 (3H,d,J=7Hz), 2.23 (3H,s), 4.41 (1H,d,J=14.6Hz), 4.97 (1H,d,J=14.6Hz), 5.06 (1H,q,J=7Hz), 5.54 (1H,s), 5.95 (1H,tt,J=53.2Hz,J=2.8Hz), 6.75-6.90 (2H,m), 7.40 (4H,s), 7.50-7.65 (1H,m), 7.70 (1H,s), 7.96 (1H,s).

Example 7

Preparation of 2-[(1R,2R)-2-(2,4-difluorophenyl)-2-hydroxy-1-methyl-3-(1H-1,2,4-triazol-1-yl)propyl]-4-[4-(2,2,2-trifluoroethoxy)phenyl]-3(2H,4H)-1,2,4-triazolone.

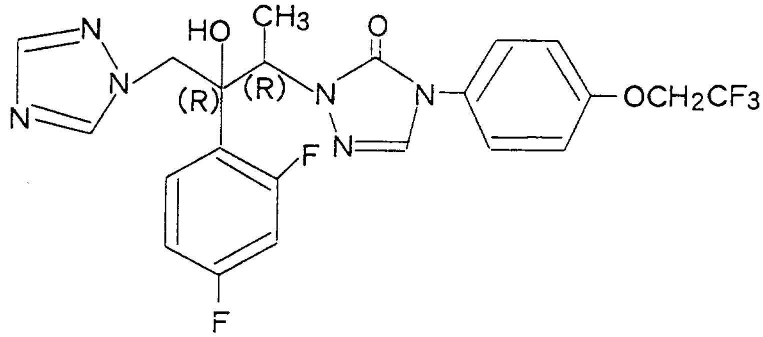

(2R,3S)-2-(2,4-Difluorophenyl)-3-methyl-2-[(1H-1,2,4-triazol-1-yl)methyl]oxirane (2.5 g), 4-[4-(2,2,2-trifluoroethoxy)phenyl]-3(2H,4H)-1,2,4-triazolone (3.88 g) and potassium carbonate (powder: 6.9 g) were added to 50 ml of N,N-dimethylformamide, and the resulting mixture was stirred at 70 °C for 54 hours. The reaction solution was cooled, diluted with 200 ml of ethyl acetate, and washed with water (50 ml), 0.25N-sodium hydroxide water (50 ml X 3), 1N-hydrochloric acid (50 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; hexane:ethyl acetate = 2:3 to 1:4) and crystallized from ethyl acetate-diisopropyl ether (1:3, 30 ml) to give

the title compound (3.89 g, yield: 76 %) as colorless needles.
mp 168-169 °C

| Elemental analysis for $C_{22}H_{19}F_5N_6O_3$ | | | |
|---|---|---|---|
| Calcd: | C 51.77, | H 3.75, | N 16.46 |
| Found: | C 51.79, | H 3.76, | N 16.53 |

$^1$H-NMR ($CDCl_3$) δ: 1.30 (3H,d,J = 7Hz), 4.35 (1H,d,J = 14.8Hz), 4.40 (2H,q,J = 8Hz), 5.02 (1H,d,J = 14.8Hz), 5.08 (1H,q,J = 7Hz), 5.47 (1H,s), 6.74-6.89 (2H,m), 7.08 (2H,d,J = 9Hz), 7.48-7.65 (1H,m), 7.53 (2H,d,J = 9Hz), 7.68 (1H,s), 7.75 (1H,s), 7.94(1H,s).

Example 8

Preparation of 2-[(1R,2R)-2-(2,4-difluorophenyl)-2-hydroxy-1-methyl-3-(1H-1,2,4-triazol-1-yl)propyl]-4-[4-(1,1,2,2-tetrafluoroethoxy)phenyl]-3(2H,4H)-1,2,4-triazolone.

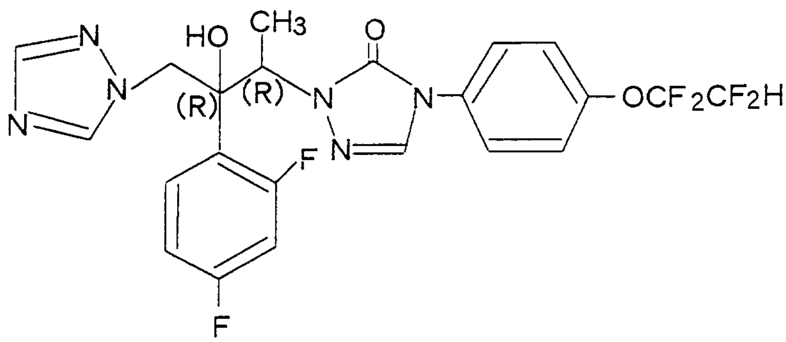

(2R,3S)-2-(2,4-Difluorophenyl)-3-methyl-2-[(1H-1,2,4-triazol-1-yl)methyl]oxirane (2.5 g), 4-[4-(1,1,2,2-tetrafluoroethoxy)phenyl]-3(2H,4H)-1,2,4-triazolone (4.1 g) and potassium carbonate (powder: 4.2 g) were added to 50 ml of N,N-dimethylformamide, and the resulting mixture was stirred at 70 °C for 60 hours and then at 100 °C for 9 hours. The reaction mixture was cooled, diluted with 300 ml of ethyl acetate, and washed with water (100 ml), 1N-sodium hydroxide water (100 ml x 3), 1N-hydrochloric acid (100 ml x 2) and saturated aqueous solution of sodium chloride (100 ml) successively. The ethyl acetate layer was dried over anhydrous magnesium sulfate and concentrated under reduced pressure. The residue was purified by silica gel chromatography (eluate ; hexane:ethyl acetate = 2:3 to 1:3) and crystallized from diisopropyl ether (30 ml) to give the title compound (2.94 g, yield: 56 %) as colorless crystals.

The title compound was recrystallized from a mixture of ethanol and water to give colorless needles.
mp 121 - 123 °C

| Elemental analysis for $C_{22}H_{18}F_6N_6O_3$ | | | |
|---|---|---|---|
| Calcd: | C 50.01, | H 3.43, | N 15.90 |
| Found: | C 49.84, | H 3.46, | N 16.25 |

$^1$H-NMR ($CDCl_3$) δ: 1.31 (3H,d,J = 7Hz), 4.37 (1H,d,J = 14.4Hz), 5.03 (1H,d,J = 14.4Hz), 5.09 (1H,q,J = 7Hz), 5.41 (1H,s), 5.94 (1H,tt,J = 53Hz,J = 2.8Hz), 6.75-6.90 (2H,m), 7.38 (2H,d,J = 9Hz), 7.50-7.70 (1H,m), 7.63 (2H,d,J = 9Hz), 7.69 (1H,s), 7.80 (1H,s), 7.94(1H,s).

Example 9

Preparation of 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

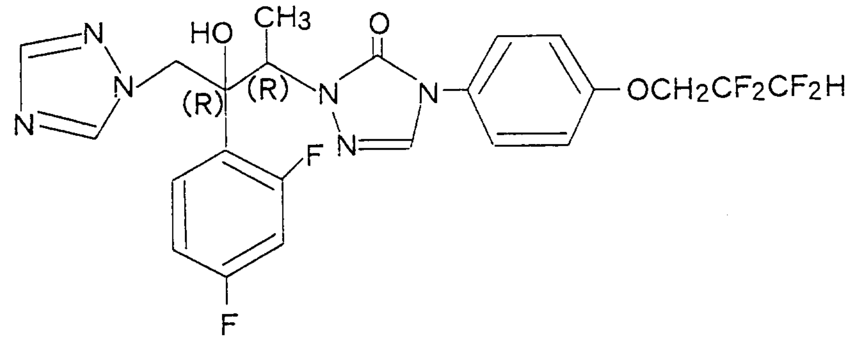

(2R,3S)-2-(2,4-Difluorophenyl)-3-methyl-2-[(1H-1,2,4-triazol-1-yl)methyl]oxirane (0.5 g), 4-[4-(2,2,3,3-tetrafluoropropoxy)phenyl]-3(2H,4H)-1,2,4-triazolone (0.83 g) and potassium hydrogencarbonate (powder: 2 g) were added to 5 ml of N,N-dimethylformamide, and the resulting mixture was heated at 80 °C for 39 hours with stirring. The reaction mixture was cooled and diluted with 20 ml of ethyl acetate. Ice water (20 ml) was added to separate the ethyl acetate layer. The aqueous layer was extracted with 10 ml of ethyl acetate. The ethyl acetate layers were combined and washed with 0.5N-sodium hydroxide water (15 ml x 3), 1N-hydrochloric acid (15 ml x 1) and saturated aqueous solution of sodium chloride (15 ml) successively. The ethyl acetate layer was dried over anhydrous magnesium sulfate and concentrated under reduced pressure. The residue was purified by silica gel chromatography (eluate ; ethyl acetate:dichloromethane = 1:1) and crystallized from diisopropyl ether to give the title compound (0.61 g, gross yield: 56 %) as colorless crystals.

It was confirmed that the compound obtained above was identical to the compound prepared in Example 5 from the mp and NMR data.

Example 10

Preparation of 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

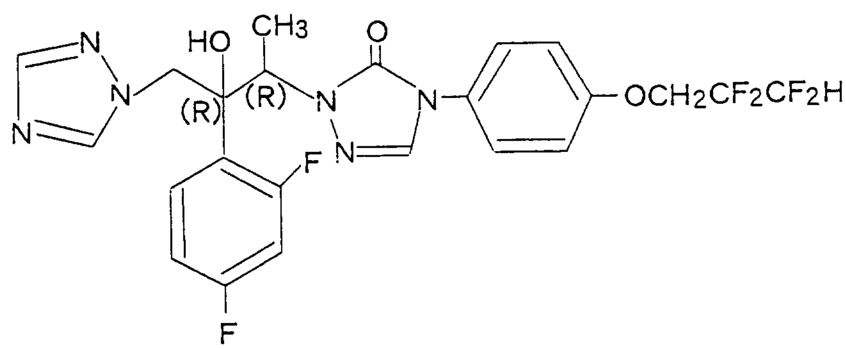

(2R,3S)-2-(2,4-Difluorophenyl)-3-methyl-2-[(1H-1,2,4-triazol-1-yl)methyl]oxirane (0.5 g), 4-[4-(2,2,3,3-tetrafluoroethoxy)phenyl]-3(2H,4H)-1,2,4-triazolone (0.83 g) and lithium hydroxide (powder: 0.84 g) were added to 5 ml of N,N-dimethylformamide, and the resulting mixture was stirred at 80 °C for 39 hours. The reaction mixture was cooled and diluted with 20 ml of ethyl acetate and 20 ml of ice water to separate the ethyl acetate layer. The aqueous layer was extracted with ethyl acetate (10 ml X 3). The ethyl acetate layers were combined, and washed with 0.5N-sodium hydroxide water (15 ml X 3), 1N-hydrochloric acid (15 ml X 1) and saturated aqueous solution of sodium chloride (15 ml) successively. The ethyl acetate layers were dried over anhydrous magnesium sulfate and concentrated under reduced pressure. The residue was purified by silica gel chromatography (eluate ; ethyl acetate:dichloromethane = 1:1) and crystallized from diisopropyl ether to give the title compound (0.49 g, yield: 45 %) as colorless crystals.

It was confirmed that the compound obtained above was identical to the compound prepared in Example 5 from the mp and NMR data.

Example 11

Preparation of 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

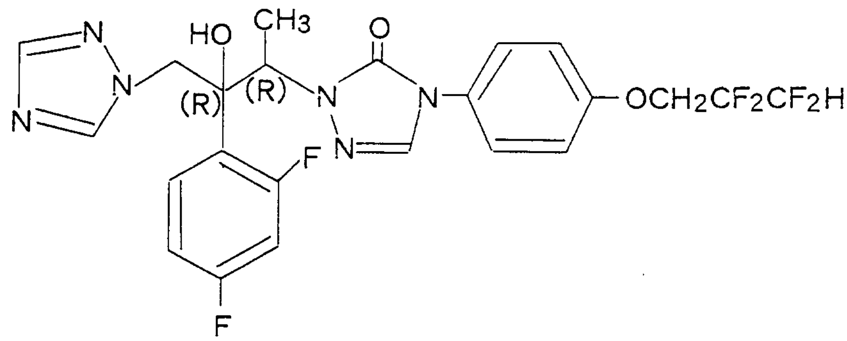

(2R,3R)-2-(2,4-Difluorophenyl)-3-(p-toluenesulfonyloxy) -1-(1H-1,2,4-triazol-1-yl)-2-butanol (0.9 g), 4-[4-(2,2,3,3-tetrafluoropropoxy)phenyl]-3(2H,4H)-1,2,4-triazolone (0.87 g) and potassium carbonate (powder: 1.46 g) were added to 18 ml of N,N-dimethylformamide, and the resulting mixture was stirred at 80 °C for 44 hours. The reaction mixture was cooled and diluted with 100 ml of ethyl acetate. Ice water (200 ml) was added to separate the ethyl acetate layer. The aqueous layer was extracted with ethyl acetate (50 ml x 2). The ethyl acetate layers were combined and washed with 0.5N-sodium hydroxide water (15 ml x 2), 1N-hydrochloric acid (50 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; dichloromethane:ethyl acetate = 1:1) and crystallized from diisopropyl ether to give the title compound (0.53 g, yield: 46 %) as colorless crystals.

It was confirmed that the compound obtained above was identical to the compound prepared in Example 5 from the mp and NMR data.

Example 12

Preparation of 4-[(1R,2R)-2-(2,4-difluorophenyl)-2-hydroxy-1-methyl-3-(1H-1,2,4-triazol-1-yl)propyl]-2-[4-(1,1,2,2-tetrafluoroethoxy)phenyl]-3(2H,4H)-1,2,4-triazolone.

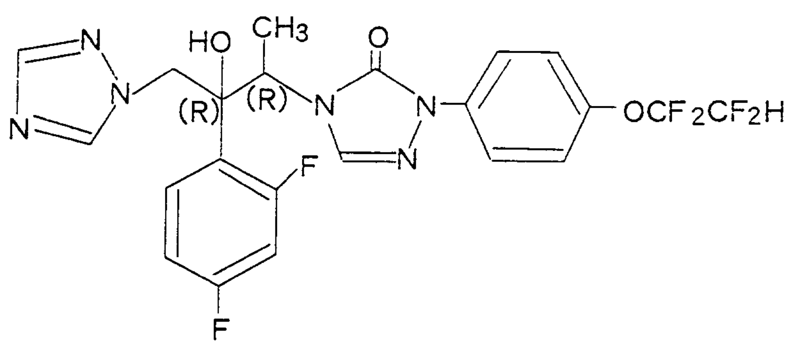

(2R,3R)-2-(2,4-Difluorophenyl)-3-(p-toluene sulfonyloxy)-1-(1H-1,2,4-triazol-1-yl)-2-butanol (1 g), 2-[4-(1,1,2,2-tetrafluoroethoxy)phenyl]-3(2H,4H)-1,2,4-triazolone (0.92 g) and potassium carbonate (powder: 1.63 g) were added to 20 ml of N,N-dimethylformamide, and the resulting mixture was heated at 80 °C for 52 hours with stirring. The reaction mixture was cooled and diluted with 100 ml of ethyl acetate. Ice water (200 ml) was added to separate the ethyl acetate layer. The aqueous layer was extracted with ethyl acetate (50 ml x 2). The ethyl acetate layers were combined and washed with 0.5N-sodium hydroxide water (50 ml x 2), 1N-hydrochloric acid (50 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; ethyl acetate:hexane = 1:1) and crystallized

from ethyl acetate - hexane (1:5) to give the title compound (0.48 g, yield: 38 %) as colorless crystals.

It was confirmed that the compound obtained above was identical to the compound prepared in Example 3 from the mp and NMR data.

Example 13

Preparation of 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

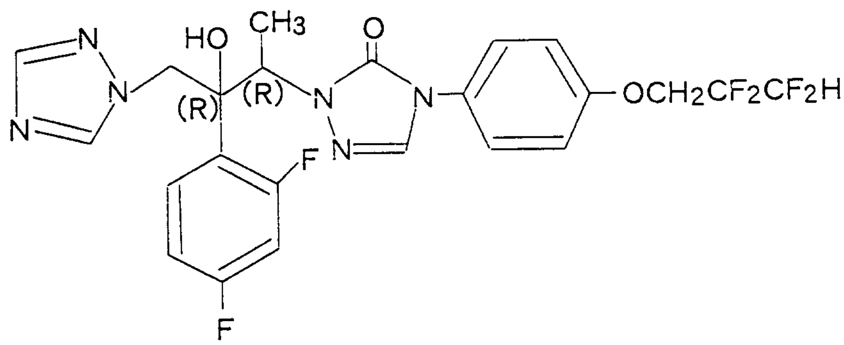

(2R,3R)-2-(2,4-Difluorophenyl)-3-methanesulfonyloxy-1-(1H-1,2,4-triazol-1-yl)-2-butanol (2 g), 4-[4-(2,2,3,3-tetrafluoropropoxy)phenyl]-3(2H,4H)-1,2,4-triazolone (2.51 g) and potassium carbonate (powder: 3.95 g) were added to 40 ml of N,N-dimethylformamide, and the resulting mixture was heated at 80 °C for 24 hours with stirring and then at 100 °C for 4 hours. The reaction mixture was cooled and diluted with 200 ml of ethyl acetate. Ice water (300 ml) was added to separate the ethyl acetate layer. The aqueous layer was extracted with ethyl acetate (100 ml x 2). The ethyl acetate layers were combined and washed with 0.5N-sodium hydroxide water (100 ml x 2), 1N-hydrochloric acid (100 ml) and saturated aqueous solution of sodium chloride (100 ml) successively. The ethyl acetate layer was dried over anhydrous magnesium sulfate and concentrated under reduced pressure. The residue was purified by silica gel chromatography (eluate ; ethyl acetate:dichloromethane = 1:1) and crystallized from diisopropyl ether to give the title compound (1.97 g, yield: 63 %) as colorless crystals.

It was confirmed that the compound obtained above was identical to the compound prepared in Example 5 from the mp and NMR data.

Example 14

Preparation of 4-[(1R,2R)-2-(2,4-difluorophenyl)-2-hydroxy-1-methyl-3-(1H-1,2,4-triazol-1-yl)propyl]-2-[4-(1,1,2,2-tetrafluoroethoxy)phenyl]-3(2H,4H)-1,2,4-triazolone.

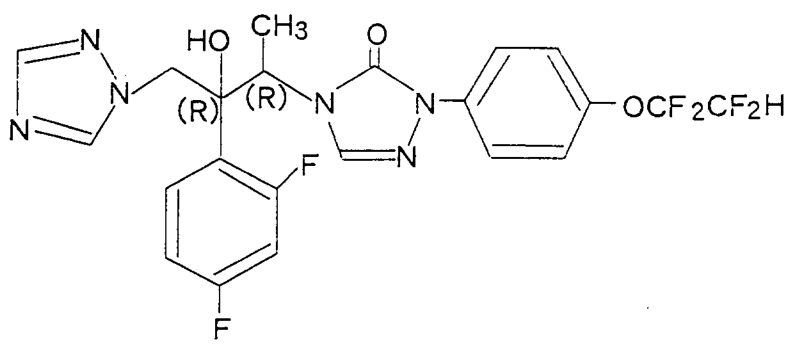

(2R,3R)-2-(2,4-Difluorophenyl)-3-methanesulfonyloxy-1-(1H-1,2,4-triazol-1-yl)-2-butanol (0.82 g), 2-[4-(1,1,2,2-tetrafluoroethoxy)phenyl]-3(2H,4H)-1,2,4-triazolone (1.3 g) and potassium carbonate (powder: 1.63 g) were added to 15 ml of N,N-dimethylformamide, and the resulting mixture was heated at 80 °C for 64 hours with stirring. The reaction mixture was cooled and diluted with 100 ml of ethyl acetate. Ice water (200

ml) was added to separate the ethyl acetate layer. The aqueous layer was extracted with ethyl acetate (50 ml x 2). The ethyl acetate layers were combined, and washed with 0.5N-sodium hydroxide water (50 ml x 2), 1N-hydrochloric acid (50 ml) and saturated aqueous solution of sodium chloride (50 ml) successively. The ethyl acetate layer was dried over anhydrous magnesium sulfate and concentrated under reduced pressure. The residue was purified by silica gel chromatography (eluate ; ethyl acetate: hexane = 1:1) and crystallized from ethyl acetate-hexane (1:5) to give the title compound (0.4 g, yield: 32 %) as colorless crystals.

It was confirmed that the compound obtained above was identical to the compound prepared in Example 3 from the mp and NMR data.

Example 15

(2R,3S)-2-(2,4-Difluorophenyl)-3-methyl-2-[(1H-1,2,4-triazol-1-yl)methyl]oxirane and 4-[4-[4-[4-(1,1,2,2-tetrafluoroethoxy)phenyl]-1-piperazinyl]phenyl]-3(2H,4H)-1,2,4-triazolone were reacted in the same manner as that described in Example 10 to give 2-[(1R,2R)-2-(2,4-difluorophenyl)-2-hydroxy-1-methyl-3-(1H-1,2,4-triazol-1-yl)propyl]-4-[4-[4-[4-(1,1,2,2-tetrafluoroethoxy)phenyl]-1-piperazinyl]phenyl]-3(2H,4H)-1,2,4-triazolone.

| Elemental analysis for $C_{32}H_{30}F_6N_8O_3$ | | | |
|---|---|---|---|
| Calcd:<br>Found: | C 55.81,<br>C 55.81, | H 4.39,<br>H 4.41, | N 16.27<br>N 16.16 |

$^1$H-NMR ($CDCl_3$) δ: 1.30 (3H,d,J = 7Hz), 3.23-3.48 (8H,m), 4.36 (1H,d,J = 14.4Hz), 5.03 (1H,d,J = 14.4Hz), 5.09 (1H,q,J = 7Hz), 5.58 (1H,s), 5.90 (1H, tt, J = 53Hz, 2.8Hz), 6.77-7.00(6H,m), 7.10 (2H,d,J = 9Hz), 7.43 (2H,d,J = 9Hz), 7.50-7.62 (1H,m), 7.69 (1H,s), 7.74(1H,s), 7.97(1H,s).

Example 16

(2R,3S)-2-(2-Fluorophenyl)-3-methyl-2-[(1H-1,2,4-triazol-1-yl)methyl]oxirane (2 g), 4-[4-(2,2,3,3-tetrafluoropropoxy)phenyl]-3(2H,4H)-1,2,4-triazolone (3.75 g) and potassium carbonate (powder: 5.93 g) were added to N,N-dimethylformamide (40 ml), and the resulting mixture was stirred at 80 °C for 21 hours and then at 100 °C for 9 hours. After cooling, the mixture was diluted with ethyl acetate (150 ml) and washed with 1N-sodium hydroxide solution (70 ml x 2), 1N-hydrochloride (70 ml x 2) and saturated aqueous solution of sodium chloride (70 ml) successively. The organic layer was dried over anhydrous magnesium sulfate and concentrated under reduced pressure. The residue was purified by silica gel chromatography (eluate; dichloromethane: acetone = 5:1) and crystallized from ethyl acetate-diisopropyl ether (1:10, 20ml) to give 2-[(1R,2R)-2-(2-fluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone (1.93 g) as colorless crystals.

| Elemental analysis for $C_{23}H_{21}F_5N_6O_3$ | | | |
|---|---|---|---|
| Calcd:<br>Found: | C 52.67,<br>C 52.72, | H 4.04,<br>H 4.05, | N 16.02<br>N 16.03 |

$^1$H-NMR ($CDCl_3$) δ: 1.30 (3H,d,J = 7Hz), 4.35-4.46 (3H,m), 5.17 (1H,d,J = 7Hz), 5.26 (1H,q,J = 7Hz), 5.34 (1H,s), 6.07 (1H, tt, J = 4.8Hz, 53Hz), 6.99-7.33(3H,m), 7.06 (2H,d,J = 9Hz), 7.49-7.75 (1H,m), 7.54 (2H,d,J = 9Hz), 7.68 (1H,s), 7.75(1H,s), 7.89(1H,s).

Example 17

(2R,3S)-2-(2-Fluorophenyl)-3-methyl-2-[(1H-1,2,4-triazol-1-yl)methyl]oxirane (1 g) and 4-(4-trifluoromethylphenyl)-3(2H,4H)-1,2,4-triazolone (1.4 g) were reacted in the same manner as that described in Example 15 to give 2-[(1R,2R)-2-(2-fluorophenyl)-2-hydroxy-1-methyl-3-(1H-1,2,4-triazol-1-yl)propyl]-4-(4-trifluoromethylphenyl)-3(2H,4H)-1,2,4-triazolone (0.48 g).

| Elemental analysis for $C_{21}H_{18}F_4N_6O_2$ | | | |
|---|---|---|---|
| Calcd: | C 54.55, | H 3.92, | N 18.17 |
| Found: | C 54.44, | H 4.00, | N 18.11 |

$^1$H-NMR ($CDCl_3$) δ: 1.31 (3H,d,J=7Hz), 4.40 (1H,d,J=14Hz), 5.07 (1H,d,J=14Hz), 5.15 (1H,q,J=7Hz), 5.24 (1H,s), 7.00-7.34(3H,m), 7.53-7.79 (5H,m), 7.62 (1H,s), 7.79 (1H,s), 7.88 (1H,s).

Example 18

(2R,3S)-2-(2-Fluorophenyl)-3-methyl-2-[(1H-1,2,4-triazol-1-yl)methyl]oxirane (0.89 g) and 4-(4-trifluoromethoxyphenyl)-3(2H,4H)-1,2,4-triazolone (1.41 g) were reacted in the same manner as that described in Example 15 to give 2-[(1R,2R)-2-(2-fluorophenyl)-2-hydroxy-1-methyl-3-(1H-1,2,4-triazol-1-yl)-propyl]-4-(4-trifluoromethoxyphenyl)-3(2H,4H)-1,2,4-triazolone (0.42 g).

| Elemental analysis for $C_{21}H_{18}F_4N_6O_3$ | | | |
|---|---|---|---|
| Calcd: | C 52.72, | H 3.79, | N 17.57 |
| Found: | C 52.76, | H 3.87, | N 17.64 |

$^1$H-NMR ($CDCl_3$) δ: 1.31 (3H,d,J=7Hz), 4.39 (1H,d,J=14Hz), 5.06 (1H,d,J=14Hz), 5.15 (1H,q,J=7Hz), 5.28 (1H,s), 7.00-7.41 (5H,m), 7.54-7.63 (1H,m), 7.65 (2H,d,J=9Hz), 7.69 (1H,s), 7.80 (1H,s), 7.88 (1H,s).

Example 19

(2R,3S)-2-(2-Fluorophenyl)-3-methyl-2-[(1H-1,2,4-triazol-1-yl)methyl]oxirane (1 g) and 4-[4-(2,2,2-trifluoroethoxy)phenyl]-3(2H,4H)-1,2,4-triazolone (1.67 g) were reacted in the same manner as that described in Example 15 to give 2-[(1R,2R)-2-(2-fluorophenyl)-2-hydroxy-1-methyl-3-(1H- 1,2,4-triazol-1-yl)-propyl]-4-[4-(2,2,2-trifluoroethoxy)phenyl]-3(2H,4H)-1,2,4-triazolone (0.77 g).

| Elemental analysis for $C_{22}H_{20}F_4N_6O_3$ | | | |
|---|---|---|---|
| Calcd: | C 53.66, | H 4.09, | N 17.07 |
| Found: | C 53.36, | H 4.14, | N 17.15 |

$^1$H-NMR ($CDCl_3$) δ: 1.30 (3H,d,J=7Hz), 4.35-4.47 (3H,m), 5.07 (1H,d,J=14Hz), 5.15 (1H,q,J=7Hz), 5.35 (1H,s), 7.00-7.32 (3H,m), 7.08 (2H,d,J=9Hz), 7.51-7.63 (1H,m), 7.54 (2H,d,J=9Hz), 7.69 (1H,s), 7.75 (1H,s), 7.90 (1H,s).

Example 20

(2R,3S)-2-(2-Fluorophenyl)-3-methyl-2-[(1H-1,2,4-triazol-1-yl)methyl]oxirane (2 g) and 4-[4-(1,1,2,2-tetrafluoroethoxy)phenyl]-3(2H,4H)-1,2,4-triazolone (3.57 g) were reacted in the same manner as that described in Example 15 to give 2-[(1R,2R)-2-(2-fluorophenyl)-2-hydroxy-1-methyl-3-(1H-1,2,4-triazol-1-yl)-propyl]-4-[4-(1,1,2,2-tetrafluoroethoxy)phenyl]-3(2H,4H)-1,2,4-triazolone (1.35 g).

| Elemental analysis for $C_{22}H_{19}F_5N_6O_3$ | | | |
|---|---|---|---|
| Calcd: | C 51.77, | H 3.75, | N 16.46 |
| Found: | C 51.54, | H 3.66, | N 16.47 |

$^1$H-NMR ($CDCl_3$) δ: 1.31 (3H,d,J=7Hz), 4.39 (1H,d,J=14Hz), 5.07 (1H,d,J=14Hz), 5.14 (1H,q,J=7Hz), 5.29 (1H,s), 5.94(1H,tt,J=2.8Hz,53Hz), 7.00-7.12 (2H,m), 7.21-7.62 (4H,m), 7.64 (2H,d,J=9Hz), 7.68 (1H,s), 7.80 (1H,s), 7.89 (1H,s).

Example 21

(2R,3S)-2-(2-Fluorophenyl)-3-methyl-2-[(1H-1,2,4-triazol-1-yl)methyl]oxirane (2.33 g) and 5-methyl-4-[4-(1,1,2,2-tetrafluoroethoxy)phenyl]-3(2H,4H)-1,2,4-triazolone (4.36 g) were reacted in the same manner as that described in Example 15 to give 2-[(1R,2R)-2-(2-fluorophenyl)-2-hydroxy-1-methyl-3-(1H-1,2,4-triazol-1-yl)propyl]-5-methyl-4-[4-(1,1,2,2-tetrafluoroethoxy)phenyl]-3(2H,4H)-1,2,4-triazolone (3.06 g).

| Elemental analysis for $C_{23}H_{21}F_5N_6O_3$ | | | |
|---|---|---|---|
| Calcd: | C 52.67, | H 4.04, | N 16.02 |
| Found: | C 52.56, | H 4.14, | N 15.99 |

$^1$H-NMR ($CDCl_3$) δ: 1.26 (3H,d,J = 7Hz), 2.23 (3H,s), 4.44 (1H,d,J = 14.4Hz), 5.01 (1H,d,J = 14.4Hz), 5.13 (1H,q,J = 7Hz), 5.41 (1H,s), 5.95(1H,tt,J = 53Hz,2.8Hz), 7.00-7.14 (2H,m), 7.20-7.35 (1H,m), 7.40 (4H,s), 7.53-7.67 (1H,m), 7.69 (1H,s), 7.91 (1H,s).

Example 22

(2R,3S)-2-(2-Fluorophenyl)-3-methyl-2-[(1H-1,2,4-triazol-1-yl)methyl]oxirane (2.33 g) and 2-[4-(1,1,2,2-tetrafluoroethoxy)phenyl]-3(2H,4H)-1,2,4-triazolone (5.54 g) were reacted in the same manner as that described in Example 15 to give 4-[(1R,2R)-2-(2-fluorophenyl)-2-hydroxy-1-methyl-3-(1H-1,2,4- triazol-1-yl)-propyl]-2-[4-(1,1,2,2-tetrafluoroethoxy)phenyl]-3(2H,4H)-1,2,4-triazolone (2.6 g).

| Elemental analysis for $C_{22}H_{19}F_5N_6O_3$ | | | |
|---|---|---|---|
| Calcd: | C 51.77, | H 3.75, | N 16.46 |
| Found: | C 51.67, | H 3.75, | N 16.52 |

$^1$H-NMR ($CDCl_3$) δ: 1.29 (3H,d,J = 7Hz), 4.14 (1H,d,J = 14Hz), 5.01 (1H,q,J = 7Hz), 5.13 (1H,d,J = 14Hz), 5.43 (1H,s), 5.93(1H,tt,J = 2.8Hz,53Hz), 6.99-7.09(2H,m), 7.21-7.43 (4H,m), 7.75 (2H,s), 7.98 (1H,s), 8.07 (2H,d,J = 9Hz).

Example 23

(2R,3S)-2-(2-Fluorophenyl)-3-methyl-2-[(1H-1,2,4-triazol-1-yl)methyl]oxirane (1.99 g) and 2-[4-(2,2,3,3-tetrafluoropropoxy)phenyl]-3(2H,4H)-1,2,4-triazolone (3.74 g) were reacted in the same manner as that described in Example 15 to give 4-[(1R,2R)-2-(2-fluorophenyl)-2-hydroxy-1-methyl-3-(1H-1,2,4- triazol-1-yl)-propyl]-2-[4-(2,2,3,3-tetrafluoropropoxy)phenyl]-3(2H,4H)-1,2,4-triazolone (2.1 g).

| Elemental analysis for $C_{23}H_{21}F_5N_6O_3$ | | | |
|---|---|---|---|
| Calcd: | C 52.67, | H 4.04, | N 16.02 |
| Found: | C 52.48, | H 4.11, | N 16.04 |

$^1$H-NMR ($CDCl_3$) δ: 1.24 (3H,d,J = 7Hz), 4.16 (1H,d,J = 14Hz), 4.38 (2H,t,J = 12Hz), 5.02 (1H,q,J = 7Hz), 5.14 (1H,d,J = 14Hz), 5.43(1H,s), 6.09(1H,tt,J = 4.8Hz,53Hz), 7.02 (2H,d,J = 9Hz), 7.03-7.13 (1H,m), 7.21-7.48 (3H,m), 7.76 (2H,s), 7.96 (1H,s), 7.97 (2H,d,J = 9Hz).

Example 24

(2R,3S)-2-(4-Fluorophenyl)-3-methyl-2-[(1H-1,2,4-triazol-1-yl)methyl]oxirane (2.34 g), 4-[4-(2,2,3,3-tetrafluoropropoxy)phenyl]-3(2H,4H)-1,2,4-triazolone (4.37 g) and potassium carbonate (6.91 g, powder) were added to 50 ml of N,N-dimethylformamide, and the resulting mixture was stirred at 80 °C for 20 hours and then at 100 °C for 2 hours. After cooling, the reaction mixture was diluted with 200 ml of ethyl acetate, and washed with water (200 ml x 2), 1N-sodium hydroxide (200 ml x 2), 1N-hydrochloride (200 ml x 1) and saturated aqueous solution of sodium chloride (200 ml) successively. The organic layer was dried over anhydrous magnesium sulfate and then distilled off under reduced pressure. The residue was purified by

silica gel column chromatography (eluate; ethyl acetate:hexane = 3:1 to ethyl acetate) and crystallized from dichloromethane-diisopropyl ether (2:1, 21 ml) to give 3.12 g of 2-[(1R,2R)-2-(4-fluorophenyl)-2-hydroxy-1-methy1-3-(1H-1,2,4-triazol-1-yl)propyl]-4-[4-(2,2,3,3-tetrafluoropropoxy)phenyl]-3(2H,4H)-1,2,4-triazolone as colorless crystals.

| Elemental analysis for $C_{23}H_{21}F_5N_6O_3$ | | | |
|---|---|---|---|
| Calcd: | C 52.67, | H 4.04, | N 16.02 |
| Found: | C 52.60, | H 4.03, | N 16.29 |

$^1$H-NMR ($CDCl_3$) δ: 1.28 (3H,d,J=7Hz), 4.38(1H,d,J=14Hz), 4.41(2H,t,J=12Hz), 4.70(1H,d,J=14Hz), 4.84(1H,q,J=7Hz), 5.28(1H,s), 6.07 (1H,tt,J=53Hz,4.6Hz), 6.98-7.08(2H,m), 7.08(2H,d,J=9Hz), 7.38-7.46-(2H,m), 7.52(2H,d,J=9Hz), 7.72(1H,s), 7.73(1H,s), 7.84(1H,s).

Example 25

(2R,3S)-2-(4-Fluorophenyl)-3-methyl-2-[(1H-1,2,4-triazol-1-yl)methyl]oxirane (2 g) and 4-[4-(1,1,2,2-tetrafluoroethoxy)phenyl]-3(2H,4H)-1,2,4-triazolone (3.57 g) were reacted in the same manner as that described in Example 23 to give 2-[(1R,2R)-2-(4-fluorophenyl)-2-hydroxy-1-methyl-3-(1H-1,2,4-triazol-1-yl)-propyl]-4-[4-(1,1,2,2-tetrafluoroethoxy)phenyl]-3(2H,4H)-1,2,4-triazolone (1.54 g).

| Elemental analysis for $C_{22}H_{19}F_5N_6O_3$ | | | |
|---|---|---|---|
| Calcd: | C 51.77, | H 3.75, | N 16.46 |
| Found: | C 51.70, | H 3.68, | N 16.52 |

$^1$H-NMR ($CDCl_3$) δ: 1.29 (3H,d,J=7Hz), 4.39 (1H,d,J=14Hz), 4.70 (1H,d,J=14Hz) 4.84 (1H,q,J=7Hz), 5.23 (1H,s), 5.95 (1H,tt,J=2.6Hz,53Hz), 6.99-7.07 (2H,m), 7.33-7.46 (4H,m), 7.62 (2H,d,J=9Hz), 7.69 (1H,s), 7.78 (1H,s), 7.84 (1H,s).

Example 26

(2R,3S)-2-(4-Fluorophenyl)-3-methyl-2-[(1H-1,2,4-triazol-1-yl)methyl]oxirane (2.33 g) and 5-methyl-4-[4-(1,1,2,2-tetrafluoroethoxy)phenyl]-3(2H,4H)-1,2,4-triazolone (4.36 g) were reacted in the same manner as that described in Example 23 to give 2-[(1R,2R)-2-(4-fluorophenyl)-2-hydroxy-1-methyl-3-(1H-1,2,4-triazol-1-yl )propyl]-5-methyl-4-[4-(1,1,2,2-tetrafluoroethoxy)phenyl]-3(2H,4H)-1,2,4-triazolone (3.50 g).

| Elemental analysis for $C_{23}H_{21}F_5N_6O_3$ | | | |
|---|---|---|---|
| Calcd: | C 52.67, | H 4.04, | N 16.02 |
| Found: | C 52.25, | H 4.42, | N 15.92 |

$^1$H-NMR ($CDCl_3$) δ: 1.23 (3H,d,J=7Hz), 2.21 (3H,s), 4.42 (1H,d,J=14.2Hz), 4.65 (1H,d,J=14.2Hz), 4.81 (1H,q,J=7Hz), 5.31 (1H,s), 5.96 (1H,tt,J=53Hz,2.8Hz), 6.97-7.12 (2H,m), 7.35-7.54 (6H,m), 7.72 (1H,s), 7.86 (1H,s).

Example 27

(2R,3S)-2-(4-Fluorophenyl)-3-methyl-2-[(1H-1,2,4-triazol-1-yl)methyl]oxirane (2.34 g) and 2-[4-(1,1,2,2-tetrafluoroethoxy)phenyl]-3(2H,4H)-1,2,4-triazolone (5.54 g) were reacted in the same manner as that described in Example 23 to give 4-[(1R,2R)-2-(4-fluorophenyl)-2-hydroxy-1-methyl-3-(1H-1,2,4-triazol-1-yl)-propyl]-2-[4-(1,1,2,2-tetrafluoroethoxy)phenyl]-3(2H,4H)-1,2,4-triazolone (2.60 g).

| Elemental analysis for $C_{22}H_{19}F_5N_6O_3$ | | | |
|---|---|---|---|
| Calcd: | C 51.77, | H 3.75, | N 16.46 |
| Found: | C 51.73, | H 3.70, | N 16.63 |

$^1$H-NMR ($CDCl_3$) δ: 1.23 (3H,d,J = 7Hz), 4.16 (1H,d,J = 14Hz), 4.68-4.75 (1H,m), 4.70 (1H,d,J = 14Hz), 5.47 (1H,s), 5.93 (1H,tt,J = 53Hz,2.8Hz), 7.01 (2H,t,J = 9Hz), 7.27-7.39 (2H,m), 7.31 (2H,d,J = 9Hz),7.63 (1H,s), 7.80(1H,s), 7.97 (1H,s), 8.06 (2H,d,J = 9Hz).

Example 28

(2R,3S)-2-(4-Fluorophenyl)-3-methyl-2-[(1H-1,2,4-triazol-1-yl)methyl]oxirane (2.66 g) and 2-[4-(2,2,3,3-tetrafluoropropoxy)phenyl]-3(2H,4H)-1,2,4-triazolone (5 g) were reacted in the same manner as that described in Example 23 to give 4-[(1R,2R)-2-(4-fluorophenyl)-2-hydroxy-1-methyl-3-(1H-1,2,4-triazol-1-yl)-propyl]-2-[4-(2,2,3,3-tetrafluoropropoxy)phenyl]-3(2H,4H)-1,2,4-triazolone (4.12 g).

| Elemental analysis for $C_{23}H_{21}F_5N_6O_3$ | | | |
|---|---|---|---|
| Calcd: | C 52.67, | H 4.04, | N 16.02 |
| Found: | C 52.77, | H 3.98, | N 16.07 |

$^1$H-NMR ($CDCl_3$) δ: 1.23 (3H,d,J = 7Hz), 4.16 (1H,d,J = 14Hz), 4.38 (2H,t,J = 12Hz), 4.66-4.73 (2H,m), 5.46 (1H,s), 6.08 (1H,tt,J = 4.8Hz,53Hz), 6.96-7.04 (4H,m), 7.31-7.38 (2H,m), 7.62 (1H,s), 7.79 (1H,s), 7.93 (1H,s), 7.94 (2H,d,J = 9Hz).

Example 29

(2R,3S)-2-(2,4-Difluorophenyl)-3-methyl-2-[(1H-1,2,4-triazol-1-yl)methyl]oxirane (0.502 g), 0.87 g of 5-methyl-2-[4-(1,1,2,2-tetrafluoroethoxy)phenyl]-3(2H,4H)-1,2,4-triazolone and 1.38 g (powder) of potassium carbonate were added to 10 ml of N,N-dimethylformamide. The resulting mixture was stirred at 80 ° C for 26 hours and then at 100 ° C for 8 hours. After cooling, the reaction mixture was diluted with ethyl acetate (80 ml) and washed with water (20 ml), 0.5N-sodium hydroxide (20 ml), 1N-hydrochloride (20 ml) and saturated aqueous solution of sodium chloride (20 ml) successively. The ethyl acetate layer was dried over anhydrous magnesium sulfate and concentrated under reduced pressure. The residue was purified by silica gel column chromatography (eluate: dichloromethane:acetone = 8:1 to 6:1) to give 0.78 g of 4-[(1R,2R)-2-(2,4-difluorophenyl)-2-hydroxy-1-methyl-3-(1H-1,2,4-triazol-1-yl)propyl]-5-methyl-2-[4-(1,1,2,2-tetrafluoroethoxy)-phenyl]-3(2H,4H)-1,2,4-triazolone as a colorless powder.

| Elemental analysis for $C_{23}H_{20}F_6N_6O_3$ | | | |
|---|---|---|---|
| Calcd (%): | C 50.93, | H 3.72, | N 15.49 |
| Found (%): | C 50.53, | H 3.81, | N 15.22 |

The protective effects of the compound of the present invention against the experimental infection in mice are shown in the following Table 1.

Test Method: Five-week-old Crj:$CDF_1$ mice were inoculated with the minimum lethal dose of ***Candida albicans*** TA intravenously. The test compound was suspended in a solution of 0.5% sodium carboxymethylcellulose (CMC). The suspension was administered orally once immediately after infection. The activity was expressed in terms of $ED_{50}$ values calculated by the Reed and Muench method from the survival rate 7 days after infection.

TABLE 1

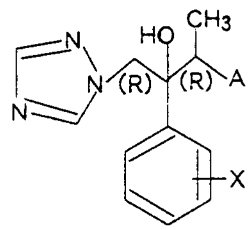

| Ex. No. | X | A | ED50 (mg/kg) |
|---|---|---|---|
| 15 | 2,4-F2 | 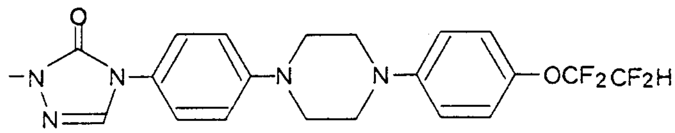 | 2.8 |
| 16 | 2-F | 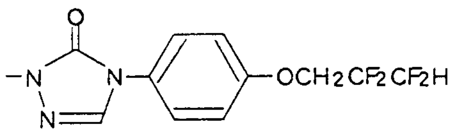 | 0.25 |
| 17 | 2-F | 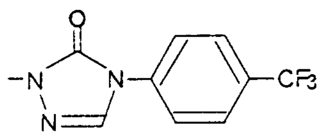 | 0.22 |
| 18 | 2-F | 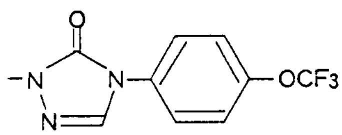 | 0.18 |
| 19 | 2-F | 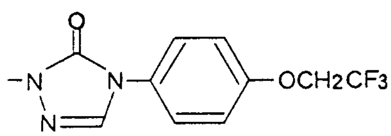 | 0.18 |
| 20 | 2-F | 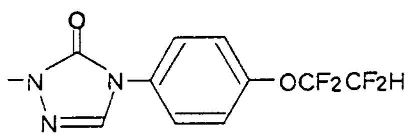 | 0.18 |
| 21 | 2-F | 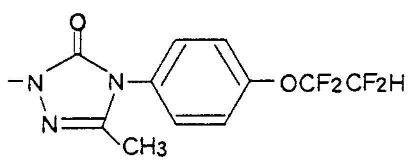 | 0.21 |

Table 1 cont'd.

| Ex. No. | X | A | ED50 (mg/kg) |
|---|---|---|---|
| 22 | 2-F | 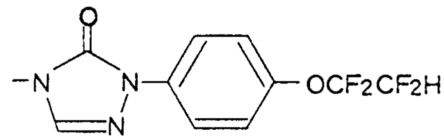 | <0.13 |
| 23 | 2-F | 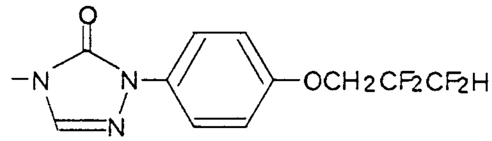 | 0.16 |
| 25 | 4-F | 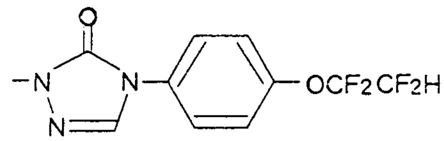 | 0.65 |
| 26 | 4-F | 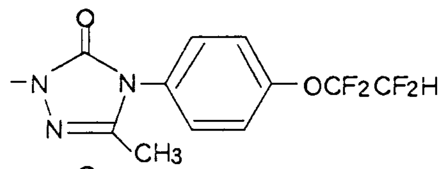 | 0.35 |
| 27 | 4-F | 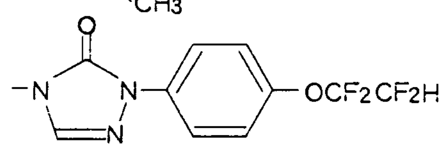 | 0.56 |
| 28 | 4-F | 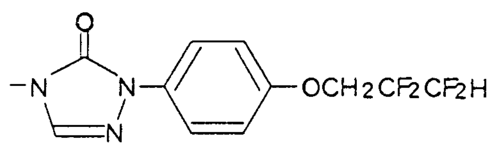 | 0.71 |
| 29 | 2,4-$F_2$ | 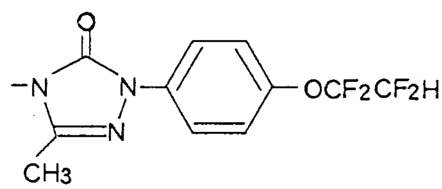 | 2.0 |

According to the process of the present invention, optically active azole derivatives which are useful for an antifungal therapeutic agent can be manufactured in a short period, by simple processes and with high yield and purity.

## Claims

**1.** A process for preparing a compound of the formula (IV) or a salt thereof:

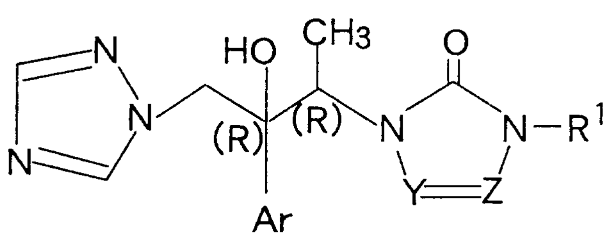

[IV]

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar is a halogenated phenyl group, and (R) shows that the carbon atom marked with (R) has (R)-configuration,

which comprises reacting a compound of the formula (I) or a salt thereof:

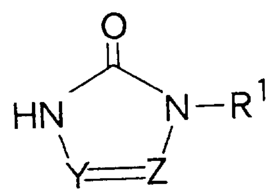

(I)

, wherein the symbols have the same meanings as defined above,

with a compound of the formula (III):

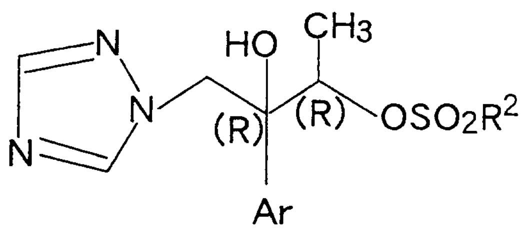

(III)

, wherein $R^2$ is a lower alkyl group or an optionally substituted phenyl group and the other symbols have the same meanings as defined above.

**2.** A process of claim 1 which is carried out in the presence of a base.

**3.** A process of claim 2 in which the base is an alkali metal carbonate, an alkali metal hydrogencarbonate, an alkali metal hydroxide, an alkali metal hydride, an alkali metal organic carboxylate, an alkali metal alcolate or tetrabutylammonium fluoride.

**4.** A process of claim 2 in which the base is an alkali metal carbonate or an alkali metal hydrogencarbonate.

**5.** A process of claim 2 in which the base is an alkali metal carbonate.

**6.** A process of claim 2 in which the base is potassium carbonate.

**7.** A process of claim 1 which is conducted in a solvent which does not impede the reaction.

**8.** A process for preparing a compound of the formula (IV) or a salt thereof:

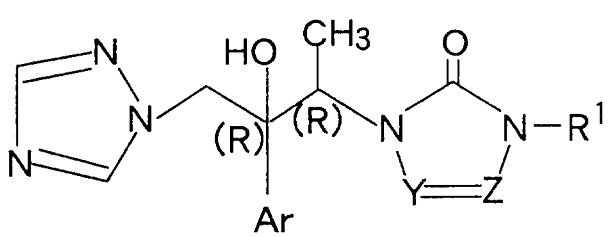

(IV)

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar is a halogenated phenyl group,

and (R) shows that the carbon atom marked with (R) has (R)-configuration,
which comprises reacting a compound of the formula (I) or a salt thereof:

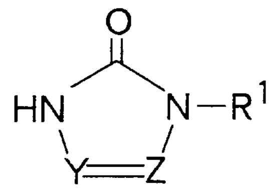

(I)

, wherein the symbols have the same meanings as defined above,
with a compound of the formula (II):

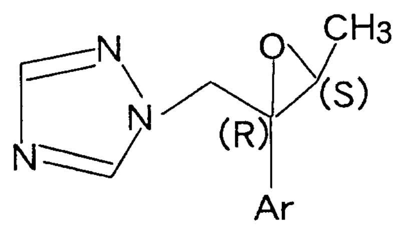

(II)

, wherein (S) shows that the carbon atom marked with (S) has (S)-configuration and the other symbols have the same meanings as defined above, in the presence of an alkali metal carbonate.

**9.** A process of claim 8 in which the alkali metal carbonate is lithium carbonate, sodium carbonate, potassium carbonate or cesium carbonate.

**10.** A process of claim 8 in which the alkali metal carbonate is potassium carbonate.

**11.** A process of claim 8 which is conducted in a solvent which does not impede the reaction.

**12.** A process of claim 11 in which the solvent is about 25 folds amount or less by weight to the compound of the formula (II).

**13.** A process of claim 1 or 8 in which the compound of the formula (IV) is 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)-phenyl]-3(2H,4H)-1,2,4-triazolone.

**14.** A process of claim 1 or 8 in which the compound of the formula (IV) is 4-[(1R,2R)-2-(2,4-difluorophenyl)-2-hydroxy-1-methyl-3-(1H-1,2,4-triazol-1-yl)propyl]-2-[4-(2,2,3,3-tetrafluoropropoxy)-phenyl]-3(2H,4H)-1,2,4-triazolone.

**15.** A process of claim 1 or 8 in which the compound of the formula (IV) is 4-[(1R,2R)-2-(2,4-difluorophenyl)-2-hydroxy-1-methyl-3-(1H-1,2,4-triazol-1-yl)propyl]-2-[4-(1,1,2,2-tetrafluoroethoxy)-phenyl]-3(2H,4H)-1,2,4-triazolone.

**16.** A process of claim 1 or 8 in which the compound of the formula (IV) is 2-[(1R,2R)-2-(2-fluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

**17.** An azole compound of the formula (IV'):

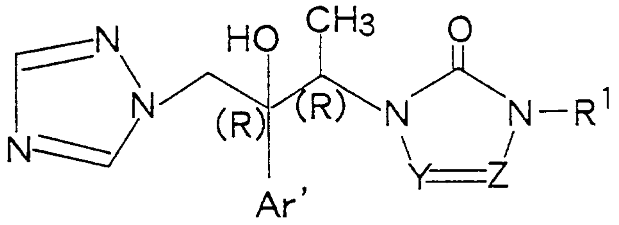

(IV')

, wherein $R^1$ is an optionally substituted aliphatic or aromatic hydrocarbon residue or an optionally substituted aromatic heterocyclic group, one of Y and Z is a nitrogen atom and the other is a methine group which may be optionally substituted with a lower alkyl group, Ar' is a monofluorophenyl group, and (R) shows that the carbon atom marked with (R) has (R)-configuration,
or a salt thereof.

**18.** A compound of claim 17 in which Ar' is 2-fluorophenyl.

**19.** A compound of claim 17 in which Ar' is 4-fluorophenyl.

**20.** A compound of claim 17 in which $R^1$ is an optionally substituted aromatic hydrocarbon residue.

**21.** A compound of claim 17 in which $R^1$ is a substituted phenyl group.

**22.** A compound of claim 17 in which $R^1$ is a phenyl group which is substituted with a haloalkoxy group.

**23.** A compound of claim 17 in which $R^1$ is 4-(2,2,3,3-tetrafluoropropoxy)phenyl.

**24.** A compound of claim 17 in which $R^1$ is 4-(1,1,2,2-tetrafluoroethoxy)phenyl.

**25.** A compound of claim 17 which is 2-[(1R,2R)-2-(2-fluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone.

**26.** Use of a compound of the formula (IV') as defined in claim 17, as an active ingredient for the preparation of an antifungal agent which comprises said compound or its pharmaceutically acceptable salt and a carrier or diluent.

European Patent Office

# EUROPEAN SEARCH REPORT

Application Number
EP 94 12 0185

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D<br>X<br><br><br><br><br>A | EP-A-0 567 982 (TAKEDA CHEMICAL INDUSTRIES, LTD.)<br>* page 2, line 19 to page 3, line 55; page 4, line 45 to page 5, line 22; page 9, line 46 to page 10, line 43; pages 62-72, 83-86; working examples 27, 49; claims 25, 30 *<br>* pages 8, 11 *<br>--- | <br><br>8,13-15, 17,26<br><br><br><br>1 | C07D403/06<br>A61K31/41 |
| A,D | EP-A-0 548 553 (TAKEDA CHEMICAL INDUSTRIES, LTD.)<br>* pages 15, 17, 18 *<br>----- | 1,8 | |
| | | | **TECHNICAL FIELDS SEARCHED (Int.Cl.6)** |
| | | | C07D |

The present search report has been drawn up for all claims

| Place of search | Date of completion of the search | Examiner |
|---|---|---|
| BERLIN | 11 April 1995 | Hass, C |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)